# NEUE EUROPÄISCHE PATENTSCHRIFT

(11) **EP 2 107 902 B2**
(45) Veröffentlichungstag und Bekanntmachung des Hinweises auf die Entscheidung über den Einspruch: **23.11.2022**
(45) Hinweis auf die Patenterteilung: 04.01.2017
(21) Anmeldenummer: 07847367.5
(22) Anmeldetag: 27.11.2007
(51) Int. Cl.: A61K 8/92, A61Q 5/12, A61Q 19/00, A61Q 5/02

(54) **KOSMETISCHE ZUSAMMENSETZUNG ENTHALTEND ARGANÖL UND SHEABUTTER**
COSMETIC COMPOSITION CONTAINING ARGAN OIL AND SHEA BUTTER
COMPOSITION COSMÉTIQUE CONTENANT DE L'HUILE D'ARGAN ET DU BEURRE DE KARITÉ

(30) Priorität: 02.01.2007 DE 102007001028
(43) Veröffentlichungstag der Anmeldung: 14.10.2009
(73) Patentinhaber: Henkel AG & Co. KGaA, 40589 Düsseldorf (DE)
(72) Erfinder: SCHULZE ZUR WIESCHE, Erik, 20144 Hamburg (DE); KURSAWE, Petra, 22527 Hamburg (DE); SOMFLETH, Petra, 21629 Neu Wulmstorf (DE); POPPE, Elisabeth, 22763 Hamburg (DE)
(74) Vertreter: LKGLOBAL Lorenz & Kopf PartG mbB Patentanwälte
(86) Internationale Anmeldenummer: PCT/EP2007/062840
(87) Internationale Veröffentlichungsnummer: WO 2008/080708

(56) Entgegenhaltungen:
- EP-A- 1 764 085
- EP-A1- 1 704 897
- EP-A1- 1 704 897
- WO-A-01/37792
- WO-A2-01/37792
- DE-U1-202004 006 865
- DE-U1-202004 006 865
- FR-A- 2 880 277
- FR-A1- 2 880 277
- JP-A- 2004 143 095
- US-A- 6 045 779
- US-A- 6 056 947
- US-A1- 2004 208 902
- US-A1- 2004 208 902
- US-A1- 2005 053 564
- US-A1- 2005 058 672
- US-A1- 2005 058 672
- US-A1- 2006 165 636
- "Stimu_tex AS - the answer to irritated and sensitive skin"[Online] 22. Oktober 2006 (2006-10-22), Seiten 1-2, XP002472942 Gefunden im Internet: URL:http://web.archive.org/web/20061022010 147/http://www.centerchem.com/PDFs/STIMU-T EX+AS+Fact+Sheet.pdf> [gefunden am 2008-03-14]
- STUSSI ET AL: "ARGANIA SPINOSA - HOW ECOLOGICAL FARMING, -AIR TRADE AND SUSTAINABILITY CAN DRIVE THE RESEARCH FOR NEW COSMETIC ACTIVE INGREDIENTS" SOFW-JOURNAL SEIFEN, OELE, FETTE, WACHSE, VERLAG FUR CHEMISCHE INDUSTRIE, AUGSBURG, DE, Bd. 131, Nr. 10, 2005, Seiten 46,48,50-52,54, XP009077275 ISSN: 0942-7694
- STIMU-TEX AS Fact Sheet; Internetveröffentlichung
- Wayback Machine; Internetveröffentlichung
- MINTEL-Veröffentlichung Nr. 807196
- MINTEL-Veröffentlichung Nr. 652198

## Beschreibung

Die Erfindung betrifft kosmetische Mittel enthaltend eine synergistisch wirksame Wirkstoffkombination enthaltend Arganöl und Sheabutter.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst lang anhaltender Wirkung zu. Derartige Pflegemittel beeinflussen die natürliche Struktur und die Eigenschaften der Haare. So können anschließend an solche Behandlungen beispielsweise die Nass- und Trockenkämmbarkeit des Haares, der Halt und die Fülle des Haares optimiert sein, sowie die äußere Struktur des Haares geglättet sein, was sich durch einen erhöhten Glanz zeigt, oder die Haare vor erhöhtem Spliss geschützt sein.

Gesucht sind als pflegende Wirkstoffe daher insbesondere bereits in kosmetischen Mitteln seit langem verwendete Inhaltsstoffe, welche sich durch eine besonders gute Verträglichkeit auszeichnen. Gleichzeitig darf sich jedoch das Wirkungsspektrum und das Eigenschaftsprofil der kosmetischen Zusammensetzung durch die Verwendung der milden und besonders gut verträglichen Inhaltsstoffe nicht gegenüber den herkömmlichen Zusammensetzungen nachteilig verändern. Dies wird weiterhin dadurch erschwert, dass in den erfindungsgemäßen Zusammensetzungen bewusst nur so wenige Inhaltsstoffe wie unbedingt notwendig verwendet werden.

Ein Inhaltsstoff, welcher in kosmetischen Zusammensetzungen prinzipiell verwendet werden kann, jedoch keinen breiten Eingang in kosmetische Zusammensetzungen gefunden hat, ist Arganöl. Arganöl selbst ist bereits seit langer Zeit bekannt und wird vielfältig vom Mensch bereits genutzt. Es gilt als das wertvollste pflanzliche Öl, weshalb es auch "Das Gold der Berber" genannt wird. Hierin liegt auch der Grund, weshalb es keine breite Verwendung gefunden hat und daher auch wenig über das Zusammenwirken mit anderen Inhaltsstoffen kosmetischer Zusammensetzungen bekannt ist.

Arganöl ist das Öl des Samens der Arganfrucht. Der Arganbaum, Argania spinosa, ist einer der ältesten Bäume der Welt. Die Fürchte mit pflaumen- oder olivenähnlichem Aussehen können nicht verzehrt werden. Der Baum kann mehrmals im Jahr Früchte tragen. Das Holz, die Blätter und die Früchte sind sehr wertvoll und werden genutzt. Die dattelgroßen Früchte werden getrocknet und gepresst. Dabei werden die Kerne der Frucht gewonnen. Die Kerne sind etwa dreimal so groß wie Haselnüsse. Die Kerne werden aufgeschlagen um daraus wiederum den Samen zu gewinnen. Diese Samen werden schließlich leicht geröstet und gemahlen um das Öl zu gewinnen. Arganöl ist leicht rötlich und hat einen walnußartigen Geschmack. Arganöl gilt als das wertvollste Pflanzenöl. Es ist ungewöhnlich reich an Tocopherolen und zeigt von allen bekannten Pflanzenölen die stärkste Vitamin E Aktivität. Weiterhin besitzt Arganöl eine einmalig hohe Konzentration an ungesättigten Fettsäuren von mehr als 80 Gew.%. Damit ist Arganöl ein hervorragend geeignetes Speiseöl und Nahrungsergänzungsmittel. Und auch in kosmetischen Zusammensetzungen wird Arganöl aufgrund seines hohen Anteiles an Vitamin E und ungesättigten Fettsäuren verwendet. Die Bedeutung des Arganöles beruht weiterhin auch auf den im Arganöl enthaltenen einzigartigen Sterole, insbesondere Schottenol und Spinasterol. Arganöl enthält an Tocopherol je 100 g Öl etwa 50 bis 90 mg an Tocopherolen, die sich wie folgt zusammensetzen: ca. 40 bis 60 mg □-Tocopherol, ca. 5 bis 15 mg ß-Tocopherol, ca. 5 bis 10 mg □-Tocopherol und etwa 0,5 bis 5 mg □-Tocopherol. Das Verhältnis von ungesättigten zu gesättigten Fettsäuren im Arganöl beträgt etwa 4,5 : 1. Die Menge an Sterolen beträgt im Arganöl etwa 120 bis 250 mg / 100 g Öl. Der Gehalt an Fettsäuren beträgt mehr als 99 Gew.%, wovon mehr als 80 Gew.% ungesättigt sind. Die Fettsäurezusammensetzung ist etwa wie folgt: Palmitinsäure 12 bis 13 %, Stearinsäure 5 bis 7 % und Arachidonsäure 0,3 bis 0,5 % sowie die ungesättigten Fettsäuren Ölsäure 43 bis 49,1 %, Linolsäure 23,9 bis 36,0 %, Linoleinsäure bis 0,1 % sowie Gadoleinsäure bis 0,5 %. Die Menge an Linolsäure ist im Arganöl etwa dreifach erhöht gegenüber dem Olivenöl.

Arganöl wird traditionell zum Kochen und in kosmetischen Zusammensetzungen verwendet. Die Nordafrikaner nutzen Arganöl zum Einreiben der Haut vor dem Besuch eines Dampfbades. Es penetriert schnell in die Haut und hinterlässt ein angenehmes und weiches Hautgefühl. Arganöl beugt der Austrocknung der Haut und der Hautalterung vor und findet auch Verwendung zur Vermeidung von Juckreiz. In der Haarpflege wird es bei sprödem und trockenem Haar sowie bei Haarausfall empfohlen.

Sheabutter ist ein weiterer Bestandteil, welcher zwar häufiger Verwendung findet als Arganöl, dessen Wirkungen in kosmetischen Zusammensetzungen jedoch ebenfalls nicht vollständig bekannt sind.

Sheabutter ist der Kakaobutter ähnlich. Sheabutter wird aus den Samen des in Westafrika vorkommenden Butterbaumes, Vitellaria paradoxa oder auch Butyrospermum parkii, Sapotacaeae, gewonnen. Sie ist von zäh-butterartiger Konsistenz und relativ stabil gegen Oxidation. In den Erzeugerländern wird Sheabutter direkt zu Nahrungszwecken verwendet. Sheabutter wird durch die folgenden Kennzahlen charakterisiert: Verseifungszahl 178 bis 196, lodzahl 55 bis 67, Unverseifbares 2 bis 11 %. Sheabutter weist einen Schmelzbereich von 32 bis 42 °C auf. Ein charakteristischer Inhaltsstoff ist das Sheasterin, 2-Oleo-distearin. Die wesentliche Fettsäurezusammensetzung ist Ölsäure 49 bis 50 %, Stearinsäure 35 bis 42 %, Palmitinsäure 5 bis 6 % und Linolsäure 4 bis 5 %. Aufgrund des hohen Anteiles an Unverseifbarem weist Sheabutter eine Sonderstellung innerhalb der pflanzlichen Öle und Fette auf. Der Anteil an Unverseifbarem beträgt üblicherweise bei pflanzlichen Fetten nur etwa 0,2 bis 2 %. Daher ist der Anteil an Phytosterolen in der Sheabutter deutlich höher als bei allen anderen pflanzlichen Fetten. Wichtige Vertreter der Phytosterole der Sheabutter sind beispielsweise α- und β-Amyrin, Basseol, Parkeol und Lupeol. Die Strukturen von α- und β-Amyrin sind wie folgt:

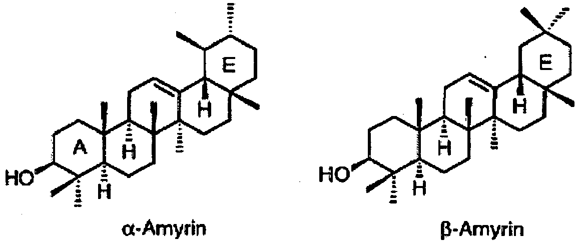

Die Struktur von Lupeol ist wie folgt:

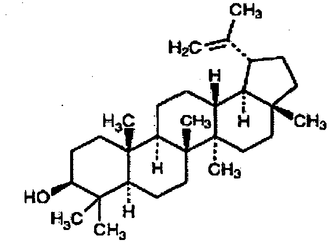

Diese Phytosterole der Sheabutter sind allesamt Triterpene mit mehr als 15 C-Atomen. Schließlich ist in der Sheabutter auch noch ein Anteil an Wachsen vorhanden.

Sheabutter wird in kosmetischen Zusammensetzungen vor allem in Pflegeprodukten für die Haut verwendet. Es ist jedoch auch in manchen Zusammensetzungen zur Reinigung und Pflege von keratinischen Fasern enthalten.

Es findet sich jedoch in keiner dem Fachmann bekannten Schriften oder Zusammensetzungen auch nur der geringste Hinweis auf das erfindungsgemäße Verfahren zur Behandlung keratinischer Fasern enthaltend eine Wirkstoffkombination aus Arganöl und Sheabutter.

Überraschenderweise wurde gefunden, dass die Verwendung einer Wirkstoffkombination enthaltend Arganöl und Sheabutter im erfindungsgemäßen Verfahren sich in ganz hervorragender Weise dazu eignet, um kosmetische Zusammensetzungen mit einer deutlich gesteigerten Verträglichkeit herzustellen. Weiterhin kann in diesen Zusammensetzungen auch aufgrund synergistischer Wirkungen zwischen den Inhaltsstoffen auf als besonders reizend bekannte Inhaltsstoffe, wie beispielsweise Parfümöle oder Konservierungsmittel ganz oder teilweise verzichtet werden. Zu den weiteren deutlich verbesserten kosmetischen Wirkungen zählen die positive Beeinflussung der Kämmbarkeit und des Griffes des nassen und trockenen Haares, des Glanzes des Haares, der Frisierbarkeit und der Haltbarkeit von Frisuren, der Restrukturierung von mit UV - Licht und/oder oxidativen Prozessen geschädigten Haaren, der Verringerung von Haarspliß, die Verringerung von Schädigungen des Haares durch Hitzeeinwirkung wie Föhnen, einer erhöhten Abscheidung von auf dem Haar erwünschten Substanzen wie Antischuppenwirkstoffen, Polymeren, Vitaminen, Proteinhydrolysaten oder weiteren das Haar avivierende Rohstoffe wie beispielsweise Silikone, die Beeinflussung des Feuchthaltevermögens der kosmetischen Zusammensetzungen, und eine die Konservierung unterstützende Wirkung in den erfindungsgemäßen kosmetischen Zusammensetzungen.

Die erfindungsgemäß verwendete Wirkstoffkombination verbessert somit signifikant in synergistischer Weise die wesentlichen inneren und äußeren Strukturmerkmale und die Festigkeit sowie die Elastizität und den Glanz von keratinischen Fasern.

In der erfindungsgemäßen Wirkstoffkombination ist Arganöl in einer Menge von 0,01 bis 30 Gew.%, bevorzugt in Mengen von 0,01 bis 20 Gew.%, besonders bevorzugt in Mengen von 0,01 bis 15 Gew.% und ganz besonders bevorzugt in Mengen von 0,01 bis 10 Gew.% jeweils bezogen auf die Wirkstoffkombination enthalten. Erfindungsgemäß ist es selbstverständlich auch möglich, anstatt des Arganöles selbst die einzelnen Bestandteile in den Mengen ihres Vorkommens im natürlichen Arganöl zu verwenden. In diesem Falle müssen jedoch die Einzelkomponenten mindestens 80 % der Zusammensetzung des natürlichen Arganöles entsprechen. Insbesondere können auf diese Weise die Fettsäuren beziehungsweise die Glyceride des Arganöles derart als Einzelkomponenten verwendet werden.

In der erfindungsgemäßen Wirkstoffkombination ist Sheabutter in einer Menge von 0,01 bis 30 Gew.%, bevorzugt in Mengen von 0,01 bis 20 Gew.%, besonders bevorzugt in Mengen von 0,01 bis 15 Gew.% und ganz besonders bevorzugt in Mengen von 0,01 bis 10 Gew.% jeweils bezogen auf die Wirkstoffkombination enthalten. Erfindungsgemäß ist es selbstverständlich auch möglich, anstatt der Sheabutter selbst die einzelnen Bestandteile in den Mengen ihres Vorkommens im natürlichen Arganöl zu verwenden. In diesem Falle müssen jedoch die Einzelkomponenten mindestens 80 % der Zusammensetzung der natürlichen Sheabutter entsprechen. Insbesondere können auf diese Weise die Fettsäuren beziehungsweise die Glyceride des Arganöles derart als Einzelkomponenten verwendet werden.

Das Verhältnis der beiden Wirkstoffe in Gewichtsprozent, Arganöl bezogen auf Sheabutter, beträgt untereinander üblicherweise 25 : 1 bis 1: 10, bevorzugt 15 : 1 bis 1 : 5, besonders bevorzugt 15 : 1 bis 1 : 3, ganz besonders bevorzugt 10 : 1 bis 1 : 3 und höchst bevorzugt 10 : 1 bis 1 : 1.

Die gesamte erfindungsgemäße Wirkstoffkombination aus Arganöl und Sheabutter wiederum ist in den kosmetischen Zubereitungen in Mengen von 0,01 bis 30 Gew.%, bevorzugt in Mengen von 0,01 bis 20 Gew.%, besonders bevorzugt in Mengen von 0,01 bis 15 Gew.% und ganz besonders bevorzugt in Mengen von 0,01 bis 10 Gew.% jeweils bezogen auf die gesamte Zusammensetzung enthalten.

Unter keratinischen Fasern werden erfindungsgemäß Pelze, Wolle, Federn und insbesondere menschliche Haare verstanden.

Ein erster Gegenstand der vorliegenden Erfindung ist daher die Verwendung einer kosmetischen Zusammensetzung zur Pflege und Konditionierung keratinischer Fasern enthaltend einen Wirkstoffkomplex, wobei der Wirkstoffkomplex Arganöl und Sheabutter enthält.

Ein zweiter Gegenstand der vorliegenden Erfindung ist weiterhin die Verwendung einer kosmetischen Zusammensetzung zur Pflege und Konditionierung keratinischer Fasern enthaltend einen Wirkstoffkomplex, wobei der Wirkstoffkomplex Arganöl und Sheabutter und mindestens einen weiteren Wirkstoff ausgewählt aus den kationischen und/oder amphoteren und/oder zwitterionischen Polymeren enthält.

Die weiteren Bestandteile der erfindungsgemäßen Zusammensetzungen werden im folgenden beschrieben. Dabei wird auf die jeweiligen synergistischen Bestandteile des erfindungsgemäßen Wirkstoffkomplexes bei der Beschreibung des jeweiligen Rohstoffes eingegangen.

Ein Inhaltsstoff, welcher in nahezu allen kosmetischen Zusammensetzungen Verwendung findet, sind oberflächenaktive Substanzen. Die oberflächenaktiven Substanzen umfassen im wesentlichen zwei Gruppen, die Tenside und die Emulgatoren.

Beispiele für besonders geeignete anionische Tenside sind, jeweils in Form der Natrium-, Kalium- und Ammonium- sowie der Mono-, Di- und Trialkanolammoniumsalze mit 2 bis 4 C-Atomen in der Alkanolgruppe,
- lineare und verzweigte Fettsäuren mit 8 bis 30 C-Atomen (Seifen),
- Ethercarbonsäuren der Formel R-O-(CH2-CH2O)x-CH2-COOH, in der R eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe, sind seit langem bekannte, hautfreundliche oberflächenaktive Stoffe, die durch Veresterung von Fettsäuren mit dem Natriumsalz der 2-Hydroxyethan-sulfonsäure (Isethionsäure), z. B. nach dem Verfahren, das in US 3,320,292 beschrieben ist, zugänglich sind. Wenn man für diese Veresterung Fettsäuren mit 8 bis 24 C-Atomen, also z. B. Laurin-, Myristin-, Palimitin- oder Stearinsäure oder auch technische Fettsäurefraktionen, z. B. die aus Kokosfettsäure erhältliche C₁₂ - C₁₈-Fettsäurefraktion einsetzt, erhält man die erfindungsgemäß bevorzugt geeigneten C₁₂ - C₁₈-Acylisethionate. Es ist bekannt, die Natriumsalze von C₁₂ - C₁₈-Acylisethionaten ähnlich wie Seifen auf Fettsäurebasis durch Kneten, Pilieren, Strangpressen, Extrudieren, Schneiden und Stückpressen in eine geeignete Form für den Transport und für die Anwendung zu bringen. Auf diese Weise lassen sich Nadeln, Granulate, Nudeln, Riegel und handliche Toilettenseifen-Stücke erzeugen.
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen. Die Sulfobernsteinsäuremonoalkyl(C₈-C₂₄)-ester-dinatriumsalze werden nach bekanntem Verfahren z. B. dadurch hergestellt, dasa man Maleinsäureanhydrid mit einem Fettalkohol mit 8 - 24 C-Atomen zum Maleinsäuremonoester des Fettalkohols umsetzt und diesen mit Natriumsulfit zum Sulfobernsteinsäureester sulfitiert. Besonders geeignete Sulfobernsteinsäureester leiten sich von Fettalkoholfraktionen mit 12 - 18 C-Atomen ab, wie sie z. B. aus Kokosfettsäure oder Kokosfettsäuremethylester durch Hydrierung zugänglich sind.
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 8 bis 30 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel (E1-I), 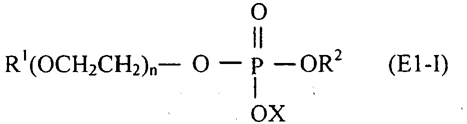
- in der R¹ bevorzugt für einen aliphatischen Kohlenwasserstoffrest mit 8 bis 30 Kohlenstoffatomen, R² für Wasserstoff, einen Rest (CH₂CH₂O)ₙR² oder X, n für Zahlen von 1 bis 10 und X für Wasserstoff, ein Alkali- oder Erdalkalimetall oder NR³R⁴R⁵R⁶, mit R³ bis R⁶ unabhängig voneinander stehend für Wasserstoff oder einen C₁ bis C₄ - Kohlenwasserstoffrest, steht,
- sulfatierte Fettsäurealkylenglykolester der Formel (E1-II)

   R⁷CO(AlkO)ₙSO₃M (E1-II)

   in der R⁷CO- für einen linearen oder verzweigten, aliphatischen, gesättigten und/oder ungesättigten Acylrest mit 6 bis 22 C-Atomen, Alk für CH₂CH₂, CHCH₃CH₂ und/oder CH₂CHCH₃, n für Zahlen von 0,5 bis 5 und M für ein Kation steht, wie sie in der DE-OS 197 36 906.5 beschrieben sind,
- Monoglyceridsulfate und Monoglyceridethersulfate der Formel (E1-III) 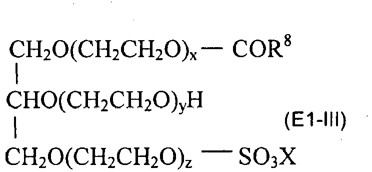
- in der R⁸CO für einen linearen oder verzweigten Acylrest mit 6 bis 22 Kohlenstoffatomen, x, y und z in Summe für 0 oder für Zahlen von 1 bis 30, vorzugsweise 2 bis 10, und X für ein Alkali- oder Erdalkalimetall steht. Typische Beispiele für im Sinne der Erfindung geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (E1-III) eingesetzt, in der R⁸C für einen linearen Acylrest mit 8 bis 18 Kohlenstoffatomen steht, wie sie beispielsweise in der EP-B1 0 561 825, der EP-B1 0 561 999, der DE-A1 42 04 700 oder von A.K.Biswas et al. in J.Am.Oil.Chem.Soc. 37, 171 (1960) und F.U.Ahmed in J.Am.Oil.Chem.Soc. 67, 8 (1990) beschrieben worden sind,
- Amidethercarbonsäuren wie sie in der EP 0 690 044 beschrieben sind,
- Kondensationsprodukte aus einem wasserlöslichen Salz eines wasserlöslichen Eiweißhydrolysat-Fettsäure-Kondensationsproduktes. Diese werden durch Kondensation von C8 - C30 Fettsäuren, bevorzugt von Fettsäuren mit 12 - 18 C-Atomen mit Aminosäuren, Mono-, Di- und wasserlöslichen Oligopeptiden und Gemischen solcher Produkte hergestellt, wie sie bei der Hydrolyse von Proteinen anfallen. Diese Eiweißhydrolysat-Fettsäure-Kondensationsprodukte werden mit einer Base neutralisiert und liegen dann bevorzugt als Alkali-, Ammonium-, Mono-, Di- oder Trialkanolammoniumsalz vor. Solche Produkte sind unter dem Warenzeichen Lamepon^{®}, Maypon^{®}, Gluadin^{®}, Hostapon^{®} KCG oder Amisoft^{®} seit langem im Handel erhältlich.

Die amphoteren und zwitterionischen Tenside zeigen eine synergistische Wirkung mit der erfindungsgemäßen Wirkstoffkombination. Diese synergistische Wirkung beruht möglicherweise auf einer verstärkten Abscheidung der beiden Wirkstoffe auf der Oberfläche von Haut und Haar, was sich im gesamten kosmetischen Erscheinungsbild von Haut und Haar bemerkbar macht. Die besonderen Ladungseffekte von amphoteren und zwitterionischen Tensiden scheinen hierbei eine Rolle zu spielen. Das Haar wird beispielsweise sowohl im trockenen wie auch im nassen Zustand besser kämmbar, leichter frisierbar und zeigt mehr Glanz sowie einen angenehmeren Griff.

Als zwitterionische Tenside (E2) werden solche oberflächenaktiven Verbindungen bezeichnet, die im Molekül mindestens eine quartäre Ammoniumgruppe und mindestens eine -COO⁽⁻⁾ - oder-SO₃⁽⁻⁾ -Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden (E3) werden solche oberflächenaktiven Verbindungen verstanden, die außer einer C₈ - C₂₄ - Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂- C₁₈ - Acylsarcosin.

Nichtionische Tenside (E4) enthalten als hydrophile Gruppe z.B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 50 Mol Ethylenoxid und/oder 0 bis 5 Mol Propylenoxid an lineare und verzweigte Fettalkohole mit 6 bis 30 C-Atomen, die Fettalkoholpolyglykolether bzw. die Fettalkoholpolypropylenglykolether bzw. gemischte Fettalkoholpolyether,
- Anlagerungsprodukte von 2 bis 50 Mol Ethylenoxid und/oder 0 bis 5 Mol Propylenoxid an lineare und verzweigte Fettsäuren mit 6 bis 30 C-Atomen, die Fettsäurepolyglykolether bzw. die Fettsäurepolypropylenglykolether bzw. gemischte Fettsäurepolyether,
- Anlagerungsprodukte von 2 bis 50 Mol Ethylenoxid und/oder 0 bis 5 Mol Propylenoxid an lineare und verzweigte Alkylphenole mit 8 bis 15 C-Atomen in der Alkylgruppe, die Alkylphenolpolyglykolether bzw. die Alkylpolypropylenglykolether, bzw. gemischte Alyklphenolpolyether,
- mit einem Methyl- oder C₂ - C₆ - Alkylrest endgruppenverschlossene Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe, wie beispielsweise die unter den Verkaufsbezeichnungen Dehydol^{®} LS, Dehydol^{®} LT (Cognis) erhältlichen Typen,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Polyolfettsäureester, wie beispielsweise das Handelsprodukt Hydagen^{®} HSP (Cognis) oder Sovermol^{®}-Typen (Cognis),
- alkoxilierte Triglyceride,
- alkoxilierte Fettsäurealkylester der Formel (E4-I)

   R¹CO-(OCH₂CHR²)_{w}OR³ (E4-I)

   in der R¹CO für einen linearen oder verzweigten, gesättigten und/oder ungesättigten Acylrest mit 6 bis 22 Kohlenstoffatomen, R² für Wasserstoff oder Methyl, R³ für lineare oder verzweigte Alkylreste mit 1 bis 4 Kohlenstoffatomen und w für Zahlen von 1 bis 20 steht,
- Aminoxide,
- Hydroxymischether, wie sie beispielsweise in der DE-OS 19738866 beschrieben sind,
- Sorbitanfettsäureester und Anlagerungeprodukte von Ethylenoxid an Sorbitanfettsäureester wie beispielsweise die Polysorbate,
- Zuckerfettsäureester und Anlagerungsprodukte von Ethylenoxid an Zuckerfettsäureester,
- Anlagerungsprodukte von Ethylenoxid an Fettsäurealkanolamide und Fettamine,
- Zuckertenside vom Typ der Alkyl- und Alkenyloligoglykoside gemäß Formel (E4-II),

   R⁴O-[G]ₚ (E4-II)

   in der R⁴ für einen Alkyl- oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden. Stellvertretend für das umfangreiche Schrifttum sei hier auf die Übersichtsarbeit von Biermann et al. in Starch/Stärke 45, 281 (1993), B. Salka in Cosm.Toil. 108, 89 (1993) sowie J. Kahre et al. in SÖFW-Journal Heft 8, 598 (1995) verwiesen.
- Zuckertenside vom Typ der Fettsäure-N-alkylpolyhydroxyalkylamide, ein nichtionisches Tensid der Formel (E4-III), 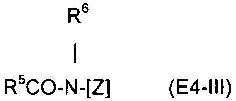 in der R⁵CO für einen aliphatischen Acylrest mit 6 bis 22 Kohlenstoffatomen, R⁶ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 12 Kohlenstoffatomen und 3 bis 10 Hydroxylgruppen steht.

Die Zuckertenside können in den erfindungsgemäß verwendeten Mitteln bevorzugt in Mengen von 0,1 - 20 Gew.-%, bezogen auf das gesamte Mittel, enthalten sein. Mengen von 0,5 - 15 Gew.-% sind bevorzugt, und ganz besonders bevorzugt sind Mengen von 0,5 - 7,5 Gew.%.

Weitere typische Beispiele für nichtionische Tenside sind Fettsäureamidpolyglycolether, Fettaminpolyglycolether, Mischether bzw. Mischformale, Proteinhydrolysate (insbesondere pflanzliche Produkte auf Weizenbasis) und Polysorbate.

Als bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure sowie die Zuckertenside erwiesen. Zubereitungen mit hervorragenden Eigenschaften werden ebenfalls erhalten, wenn sie als nichtionische Tenside Fettsäureester von ethoxyliertem Glycerin enthalten.

Diese Verbindungen sind durch die folgenden Parameter gekennzeichnet. Der Alkylrest R enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die kationischen Tenside (E5) bilden die letzte Gruppe von Tensiden. Kationische Tenside zeichnen sich als Teil des erfindungsgemäßen Wirkstoffkomplexes dadurch aus, dass sie wie die amphoteren und zwitterionischen Tenside zu einem deutlich verbesserten kosmetischen Erscheinungsbild von Haut und Haar beitragen. Die kationische Ladung sorgt für eine gute Bindung an den eher negativ geladenen Oberflächen insbesondere von geschädigtem Haar oder beanspruchter Haut. An den langen Fettresten dieser Molekülstrukturen wiederum können sich verstärkt eher hydrophob aufgebaute Wirkstoffe anlagern. Dadurch wird insgesamt eine erhöhte Abscheidung von Pflegestoffen auf der Oberfläche von Haut und Haar bewirkt. Das Haar wird beispielsweise sowohl im trockenen wie auch im nassen Zustand besser kämmbar, leichter frisierbar und zeigt mehr Glanz sowie einen angenehmeren Griff.

Kationische Tenside (E5) leiten sich im Allgemeinen von Ammoniumionen ab und besitzen eine Struktur (NR¹R²R³R⁴)⁺ mit einem entsprechend negatv geladenen Gegenion. Derartige kationische Ammoniumverbindungen sind dem Fachmann bestens bekannt. Weitere kationische Tenside sind beispielsweise die Esterquats oder die Imidazoliumverbindungen. Erfindungsgemäß besonders bevorzugt einsetzbar sind kationische Tenside (E5) vom Typ der quarternären Ammoniumverbindungen, der Esterquats, der Imidazoline und der Amidoamine. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 8 bis 30 Kohlenstoffatome auf. Typische Beispiele für kationische Tenside sind quartäre Ammoniumverbindungen und Esterquats, insbesondere quaternierte Fettsäuretrialkanolaminester-Salze.

Besonders bevorzugt einsetzbar können erfindungsgemäß kationische Verbindungen mit Behenylresten, insbesondere die unter der Bezeichnung Behentrimoniumchlorid bzw. -bromid (Docosanyltrimethylammonium Chlorid bzw. -Bromid) bekannten Substanzen. Andere bevorzugte QAV weisen mindestens zwei Behenylreste auf. Kommerziell erhältlich sind diese Substanzen beispielsweise unter der Bezeichnungen Genamin^{®} KDMP (Clariant).

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxyethyl)dimethylammoniumchlorid, sowie Dehyquart^{®} F-75, Dehyquart^{®} C-4046, Dehyquart^{®} L80 und Dehyquart^{®} AU-35 sind Beispiele für solche Esterquats.

Als weitere kationische Tenside können die erfindungsgemäßen Mittel mindestens eine quartäre Imidazolinverbindung, d.h. eine Verbindung, die einen positiv geladenen Imidazolinring aufweist, enthalten. Die im folgenden dargestellte Formel (E5-V) zeigt die Struktur dieser Verbindungen.

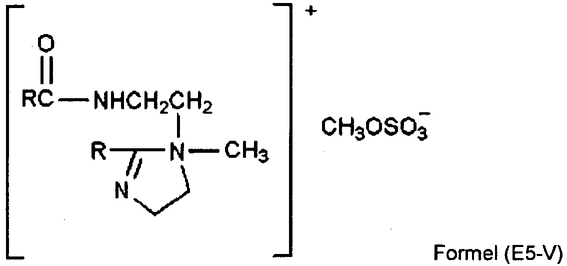

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (E5-V) enthalten für R jeweils den gleichen Kohlenwasserstoffrest. Die Kettenlänge der Reste R ist bevorzugt 12 Kohlenstoffatome. Besonders bevorzugt sind Verbindungen mit einer Kettenlänge von mindestens 16 Kohlenstoffatomen und ganz besonders bevorzugt mit mindestens 20 Kohlenstoffatomen. Eine ganz besonders bevorzugte Verbindung der Formel I weist eine Kettenlänge von 21 Kohlenstoffatomen auf. Ein Handelsprodukt dieser Kettenlänge ist beispielsweise unter der Bezeichnung Quaternium-91 bekannt. In der Formel (E5-V) ist als Gegenion Methosulfat dargestellt. Erfindungsgemäß umfasst sind jedoch als Gegenionen auch die Halogenide wie Chlorid, Fluorid, Bromid, oder auch Phosphate.

Die Imidazoline der Formel (E5-V) sind in den erfindungsgemäßen Zusammensetzungen in Mengen von 0,01 bis 20 Gew.%, bevorzugt in Mengen von 0,05 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew.% jeweils bezogen auf die Gesamtzusammensetzung des jeweiligen Mittels erhalten.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar. Die Alkylamidoamine können sowohl als solche vorliegen und durch Protonierung in entsprechend saurer Lösung in eine quaternäre Verbindung in der Zusammensetzung überführt werden, sie können aber selbstverständlich auch als permanent quaternäre Verbindung in den erfindungsgemäßen Zusammensetzungen verwendet werden. Beispiele für permanent quaternierte Amidoamine sind beispielsweise die Rohstoffe mit der Handelsbezeichnung Rewoquat^{®} UTM 50, Lanoquat^{®} DES-50 oder Empigen CSC.

Die kationischen Tenside (E5) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Kationische, zwitterionische und/oder amphotere Tenside sowie deren Mischungen können erfindungsgemäß bevorzugt sein. Anionische Tenside werden insbesondere verwendet, wenn die erfindungsgemäßen Zusammensetzungen als Shampoos verwendet werden sollen.

Die Tenside (E) werden in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Erfindungsgemäß verwendbare Emulgatoren sind beispielsweise
- Anlagerungsprodukte von 4 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Polyole mit 3 bis 6 Kohlenstoffatomen, insbesondere an Glycerin,
- Ethylenoxid- und Polyglycerin-Anlagerungsprodukte an Methylglucosid-Fettsäureester, Fettsäurealkanolamide und Fettsäureglucamide,
- C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind,
- Gemische aus Alkyl-(oligo)-glucosiden und Fettalkoholen zum Beispiel das im Handel erhältliche Produkt Montanov^{®}68,
- Anlagerungsprodukte von 5 bis 60 Mol Ethylenoxid an Rizinusöl und gehärtetes Rizinusöl,
- Partialester von Polyolen mit 3-6 Kohlenstoffatomen mit gesättigten Fettsäuren mit 8 bis 22 C-Atomen,
- Sterine. Als Sterine wird eine Gruppe von Steroiden verstanden, die am C-Atom 3 des Steroid-Gerüstes eine Hydroxylgruppe tragen und sowohl aus tierischem Gewebe (Zoosterine) wie auch aus pflanzlichen Fetten (Phytosterine) isoliert werden. Beispiele für Zoosterine sind das Cholesterin und das Lanosterin. Beispiele geeigneter Phytosterine sind Ergosterin, Stigmasterin und Sitosterin. Auch aus Pilzen und Hefen werden Sterine, die sogenannten Mykosterine, isoliert.
- Phospholipide. Hierunter werden vor allem die Glucose-Phospolipide, die z.B. als Lecithine bzw. Phospahtidylcholine aus z.B. Eidotter oder Pflanzensamen (z.B. Sojabohnen) gewonnen werden, verstanden.
- Fettsäureester von Zuckern und Zuckeralkoholen, wie Sorbit,
- Polyglycerine und Polyglycerinderivate wie beispielsweise Polyglycerinpoly-12-hydroxystearat (Handelsprodukt Dehymuls^{®} PGPH),
- Lineare und verzweigte Fettsäuren mit 8 bis 30 C - Atomen und deren Na-, K-, Ammonium-, Ca-, Mg- und Zn - Salze.

Als besonders vorteilhaft hat sich auch ein Zusatz eines an sich bekannten Emulgators vom Typ Wasser-in-Öl in einer Menge von ca. 1 - 5 Gew.-% erwiesen. Dabei handelt es sich um einen Mischester, der ein Kondensationsprodukt aus einem Pentaerythrit-di-fettsäureester und einem Zitronensäure-di-fettalkoholester darstellt, wie es in DE-PS 11 65 574 näher beschrieben ist. Durch den Zusatz solcher Mischester wird ein besonders cremiger, feinblasiger Schaum und ein angenehmes Hautgefühl bei der Anwendung des Körperreinigungsmittels erreicht.

Die erfindungsgemäßen Mittel enthalten die Emulgatoren bevorzugt in Mengen von 0,1 - 25 Gew.-%, insbesondere 0,5 - 15 Gew.-%, bezogen auf das gesamte Mittel.

In dem erfindungsgemäß bevorzugten Fall, dass die Verträglichkeit der kosmetischen Zusammensetzungen verbessert werden soll, werden die besonders milden Emulgatoren in den Zusammensetzungen bevorzugt verwendet. In diesen Fällen werden die Alkylsulfate und / oder Alkylethersulfate in Mengen unterhalb von 8 Gew.% , bevorzugt kleiner 5 Gew.% und besonders bevorzugt kleiner 2,5 Gew.% verwendet. Ganz besonders bevorzugt sind diese Zusammensetzungen frei von an Alkylsulfaten und / oder Alkylethersulfaten. "Frei von" bedeutet in diesem Zusammenhang, dass diese Inhaltsstoffe keinesfalls zusätzlich verwendet werden. Es ist jedoch möglich, dass sie durch andere Inhaltsstoffe, wie beispielsweise durch die Verwendung von Silikonemulsionen, in die Zusammensetzung gelangen. Vorzugsweise bedeutet "frei von" daher auch kleiner als 0,5 Gew.%, besonders bevorzugt kleiner 0,1 Gew.%.

Es ist bekannt, dasa Öl-in-Wasser-Emulsionen, fortan O/W-Emulsionen genannt, die mit nichtionogenen Emulgatoren hergestellt und stabilisiert sind, beim Erwärmen eine Phaseninversion erleiden, d.h., dasa bei höheren Temperaturen die äußere, wäßrige Phase zur inneren Phase werden kann. Dieser Vorgang ist in der Regel reversibel, d.h., dasa sich beim Abkühlen wieder der ursprüngliche Emulsionstyp zurückbildet. Es ist auch bekannt, dasa die Lage der Phaseninversionstemperatur (PIT) von vielen Faktoren abhängig ist, zum Beispiel von der Art und dem Phasenvolumen der Ölkomponente, von der Hydrophilie und der Struktur des Emulgators oder der Zusammensetzung des Emulgatorsystems. Weiterhin ist es bekannt, dasa Emulsionen, die bei oder wenig unterhalb der Phaseninversionstemperatur hergestellt werden, sich durch besondere Stabilität und Feinteiligkeit auszeichnen, während solche, die oberhalb der Phaseninversionstemperatur hergestellt werden, weniger feinteilig sind. Emulsionen, die bei einer bestimmten Temperatur Phaseninversion erleiden, werden PIT-Emulsionen genannt. Diese PIT-Emulsionen können erfindungsgemäß bevorzugt sein, weil sie bedingt durch die gerade hinreichende Menge an Emulgator deutlich weniger Emulgator enthalten als übliche nicht PIT-Emulsionen. Daher sind sie nicht nur besonders kostengünstig, sondern noch dazu auch besonders mild und schonend für die Haut und das Haar. Werden in den PIT-Emulsionen auch ionische Tenside als Emulgatoren verwendet, dann werden diese besonders bevorzugt erst nach der Herstellung der PIT-Emulsion während des Abkühlprozesses der PIT-Emulsion zugesetzt.

Zu den Synergisten der erfindungsgemäßen Wirkstoffkombination in den erfindungsgemäßen Zusammensetzungen zählen kationische sowie amphotere und/oder zwitterionische Polymer. Polymere werden in kosmetischen Zusammensetzungen aus einer Vielzahl von Gründen verwendet. Hierbei werden auch nichtionische Polymere verwendet.

Im folgenden werden einige Beispiele von besonders bevorzugten Polymeren beschrieben. Die Unterscheidung der erfindungsgemäß verwendbaren Polymere kann dabei aufgrund der Ladungen der Polymere und/oder aufgrund ihrer anwendungstechnischen besonders ausgeprägten Eigenschaften erfolgen. Die Ausdrucksweise "besonders ausgeprägte Eigenschaften" spiegelt den Sachverhalt wider, dass Polymere im Allgemeinen mehrere Eigenschaften in einem Molekül vereinen. Häufig steht jedoch eine der Eigenschaften ganz besonders im Vordergrund und ist für die Auswahl gerade dieses Polymeren maßgeblich.

Zunächst werden Polymere aufgrund ihrer jeweiligen Ladungen beschrieben.

Unter kationischen Polymeren sind Polymere zu verstehen, welche in der Haupt- und/oder Seitenkette eine Gruppe aufweisen, welche "temporär" oder "permanent" kationisch sein kann. Als "permanent kationisch" werden erfindungsgemäß solche Polymere bezeichnet, die unabhängig vom pH-Wert des Mittels eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C1-4-Kohlenwasserstoffgruppe an eine aus Acrylsäure, Methacrylsäure oder deren Derivaten aufgebaute Polymerhauptkette gebunden sind, haben sich als besonders geeignet erwiesen.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt.

Die erfindungsgemäßen kationischen Polymere können sowohl festigende und/oder filmbildende und/oder antistatische und/oder avivierende Polymere als auch Polymere mit konditionierenden und/oder verdickenden Eigenschaften sein. Bei den geeigneten kationaktiven Polymeren handelt es sich vorzugsweise um haarfestigende und/oder um haarkonditionierende Polymere. Unter Polymeren sind sowohl natürliche als auch synthetische Polymere, welche kationisch oder amphoter geladen sein können, zu verstehen.

Diese beiden Gruppen von Polymeren haben eine potentiell kationische Ladung gemeinsam. Sowohl kationische als auch amphotere bzw. zwitterionische Polymere können daher über ihre kationische Ladungsdichte charakterisiert werden. Die erfindungsgemäßen Polymere zeichnen sich durch eine Ladungsdichte von mindestens 1 bis 7 meq/g aus. Eine Ladungsdichte von mindestens 2 bis 7 meq/g ist dabei bevorzugt. Besonders bevorzugt ist eine Ladungsdichte von mindestens gleich 3meq/g bis 7 meq/g.

Ein weiteres charakteristisches Merkmal der erfindungsgemäßen Polymeren ist ihre Molmasse. Unter der Molmasse des jeweiligen Polymeren wird die Molmasse verstanden, welche der Hersteller in den entsprechenden Datenblättern nach dessen Methode gemessen angibt. Für die Auswahl eines geeigneten Polymeren hat sich eine Molmasse von mindestens 50.000 g/u als erfindungsgemäß geeignet erwiesen. Polymere mit einer Molmasse von mehr als 100.000 g/u haben sich als besonders geeignet erwiesen. Polymere mit einer Molmasse von mehr als 1000.000 g/u sind ganz besonders geeignet.

Die Ablagerung von Polymeren aus tensidischen Lösungen an der Oberfläche keratinischer Fasern ist ein Adsorptionsvorgang. Dieser Adsorptionsvorgang ist bis heute nicht vollständig verstanden. Die zuvor dargestellte Auswahl geeigneter Polymerer erfolgt im Stand der Technik bereits nach den zuvor dargestellten Kriterien der Ladungsdichte oder der Molmasse.

Wählt man nun die Polymere gemäß dem Stand der Technik aus, so sollten diejenigen Polymere, welche beide Kriterien erfüllen, eigentlich die geeignetsten sein. Dies ist jedoch nicht der Fall.

Alle bekannten Adsorptionsgleichungen der physikalischen Chemie enthalten Proportionalitätskonstanten, welche im Zusammenhang mit dem Belegungsgrad der Oberfläche stehen. Ohne den genauen wissenschaftlichen Hintergrund zu kennen, könnte jedoch der Belegungsgrad der Oberfläche keratinischer Fasern mit einer Adsorptionswahrscheinlichkeit der erfindungsgemäßen Polymeren zusammenhängen. Definiert man die Adsorptionswahrscheinlichkeit als das Produkt aus der kationischen Ladungsdichte und der Molmasse, so kann dieses Produkt zur gezielten Auswahl geeigneter erfinderischer Polymere herangezogen werden.

Geeignete Polymere weisen für das Produkt aus der kationischen Ladungsdichte und der Molmasse einen Wert von größer als 100.000 auf. Besonders geeignet sind Polymere, welche für dieses Produkt einen Wert von mindestens 200.000 aufweisen. Ganz besonders geeignet sind diejenigen Polymere, bei welchen dieses Produkt einen Wert größer als 250.000 aufweist. Am geeignetsten sind diejenigen Polymere, bei welchen dieses Produkt einen Wert von mindestens 1.000.000 aufweist.

Bevorzugt sind solche Polymere, die eine ausreichende Löslichkeit in Wasser oder Alkohol besitzen, um in dem erfindungsgemäßen Mittel bei der Anwendung auf dem feuchten bis nassen Haar vollständig in Lösung zu gehen.

Die kationischen Polymere können Homo- oder Copolymere sein, wobei die quaternären Stickstoffgruppen entweder in der Polymerkette oder vorzugsweise als Substituent an einem oder mehreren der Monomeren enthalten sind. Die Ammoniumgruppen enthaltenden Monomere können mit nicht kationischen Monomeren copolymerisiert sein. Geeignete kationische Monomere sind ungesättigte, radikalisch polymerisierbare Verbindungen, welche mindestens eine kationische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammoniumgruppen enthaltenden Monomere können mit nicht kationischen Monomeren copolymerisiert sein. Geeignete Comonomere sind beispielsweise Acrylamid, Methacrylamid; Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium- 11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enthalten sein können, ist zum Beispiel Vinylpyrrolidon/Dimethylaminoethylmethacrylatmethosulfat Copolymer, das unter den Handelsbezeichnungen Gafquat^{®} 755 N und Gafquat^{®} 734 von der Firma Gaf Co., USA vertrieben wird und von denen das Gafquat^{®} 734 besonders bevorzugt ist, geeignet. Weitere kationische Polymere sind beispielsweise das von der Firma BASF, Deutschland unter dem Handelsnamen Luviquat^{®} HM 550 vertriebene Copolymer aus Polyvinylpyrrolidon und Imidazoliminmethochlorid, das von der Firma Calgon/USA unter dem Handelsnamen Merquat^{®} Plus 3300 vertriebene Terpolymer aus Dimethyldiallylammoniumchlorid, Natriumacrylat und Acrylamid und das von der Firma ISP unter dem Handelsnamen Gafquat^{®} HS 100 vertriebene VinylpyrrolidonlMethacrylamidopropyltrimethylammoniumchlorid Copolymer.

Homopolymere der Allgemeinen Formel (G1-I), 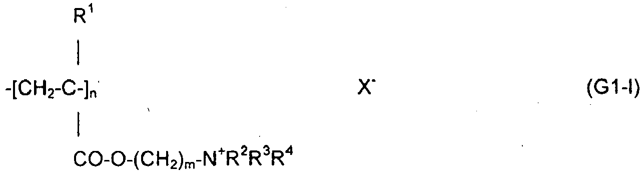 in der R¹= -H oder -CH₃ ist, R², R³ und R⁴ unabhängig voneinander ausgewählt sind aus C1-4-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel (G1-I) aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gilt:
- R¹ steht für eine Methylgruppe
- R², R³ und R⁴ stehen für Methylgruppen
- m hat den Wert 2.

Als physiologisch verträgliches Gegenionen X⁻ kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Solche Produkte sind beispielsweise unter den Bezeichnungen Rheocare^{®} CTH (Cosmetic Rheologies) und Synthalen^{®} CR (3V Sigma) im Handel erhältlich. Die Vernetzung kann gewünschtenfalls mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwässrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel (G1-I) enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwässrige Polymerdispersion unter der Bezeichnung Salcare^{®} SC 92 erhältlich.

Geeignete kationaktive Silikonverbindungen weisen vorzugsweise entweder mindestens eine Aminogruppe oder mindestens eine Ammoniumgruppe auf. Geeignete Silikonpolymere mit Aminogruppen sind unter der INCI- Bezeichnung Amodimethicone bekannt. Hierbei handelt es sich um Polydimethylsiloxane mit Aminoalkylgruppen. Die Aminoalkylgruppen können Seiten- oder endständig sein. Das N-haltige Silikon als erfindungsgemäßes kationisches Polymer kann vorzugsweise ausgewählt werden aus der Gruppe umfassend Siloxanpolymere mit wenigstens einer Aminogruppe, Siloxanpolymere mit wenigstens einer endständigen Aminogruppe, Amodimethicon, Trimethylsilylamodimethicone, und/oder Aminoethylaminopropylsiloxan-Dimethylsiloxan-Copolymer. Geeignete Silikonpolymere mit zwei endständigen quaternären Ammoniumgruppen sind unter der INCI-Bezeichnung Quaternium-80 bekannt. Hierbei handelt es sich um Dimethylsiloxane mit zwei endständigen Aminoalkylgruppen.

Erfindungsgemäß bevorzugt ist die Verwendung eines Aminosiloxans entsprechend der nachstehenden Allgemeinen Formel (G1-II), 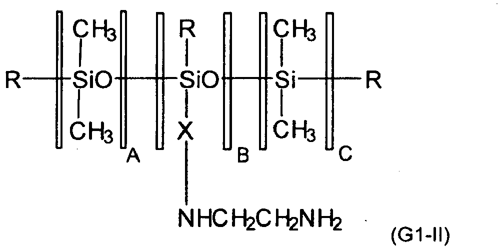 wobei R = OH oder CH₃ ; X = Alkylgruppe mit 1 bis 4 C-Atomen, vorzugsweise Propyl oder Isopropyl und A, B und C = Copolymereinheiten, die taktische und/oder ataktische Polymerblöcke ausbilden können, sind.

Erfindungsgemäß am meisten bevorzugt ist Amodimethicon, Amodimethicon haltige Emulsionen oder Fluide. Emulsionen, die erfindungsgemäß bevorzugt eingesetzt werden können sind Dow Corning^{®} 949, hierbei handelt es sich um eine kationische Emulsion enthaltend Amodimethicon, Cetrimoniumchlorid und Trideceth-12; Dow Corning^{®} 939, hierbei handelt es sich um eine Emulsion enthaltend Amodimethicon, Cetrimoniumchlorid und Trideceth-12; Dow Corning^{®} 929, hierbei handelt es sich um eine kationische Emulsion enthaltend Amodimethicon, Talktrimoniumchlorid und Nonoxynol-10; Dow Corning^{®} 7224 oder 1401, basierend auf Trimethylsilylamodimethicon, Octoxynol-40, Isolaureth-6 und Glycol; Dow Corning^{®} 2-8194 Mikroemulsion (26%ig) auf Basis eines aminfunktionalisierten Siliconpolymers; Dow Corning^{®} 2-8177 Mikroemulsion (12%ig) auf Basis eines aminfunktionalisierten Siliconpolymers; Dow Corning^{®} 2-8566 Amino Fluid auf Basis eines aminfunktionalisierten Polydimethylsiloxans; erhältlich bei der Firma Dow Corning.

Das Molekulargewicht der Aminosilikone liegt vorzugsweise zwischen 500 und 100.000. Der Aminanteil (meq/g) liegt vorzugsweise im Bereich von 0,05 bis 2,3, besonders bevorzugt von 01, bis 0,5.

Das Silikon als erfindungsgemäßes kationisches Polymer wird in einer Menge von 0,01 bis 20 Gew.% bezogen auf das gesamte Mittel, bevorzugt in Mengen von 0,05 bis 15 Gew.% und ganz besonders bevorzugt in Mengen von 0,05 bis 10 Gew.% verwendet.

Geeignete kationische Polymere, die von natürlichen Polymeren abgeleitet sind, sind kationische Derivate von Polysacchariden, beispielsweise kationische Derivate von Cellulose, Stärke oder Guar. Geeignet sind weiterhin Chitosan und Chitosanderivate. Kationische Polysaccharide haben die allgemeine Formel

(G1-III) G-O-B-N+RₐR_{b}R_{c} X⁻

G ist ein Anhydroglucoserest, beispielsweise Stärke- oder Celluloseanhydroglucose;
B ist eine divalente Verbindungsgruppe, beispielsweise Alkylen, Oxyalkylen, Polyoxyalkylen oder Hydroxyalkylen;
Rₐ, R_{b} und R_{c} sind unabhängig voneinander Alkyl, Aryl, Alkylaryl, Arylalkyl, Alkoxyalkyl oder Alkoxyaryl mit jeweils bis zu 18 C-Atomen, wobei die Gesamtzahl der C-Atome in Rₐ, R_{b} und R_{c} vorzugsweise maximal 20 ist;
X⁻ ist ein übliches Gegenanion und ist vorzugsweise Chlorid.

Eine kationische Cellulose wird unter der Bezeichnung Polymer JR^{®} 400 von Amerchol vertrieben und hat die INCI-Bezeichnung Polyquaternium-10. Eine weitere kationische Cellulose trägt die INCI-Bezeichnung Polyquaternium-24 und wird unter dem Handelsnamen Polymer LM-200 von Amerchol vertrieben. Weitere Handelsprodukte sind die Verbindungen Celquat^{®} H 100, Celquat^{®} und L 200. Die genannten Handelsprodukte sind bevorzugte kationische Cellulosen:
Geeignete kationische Guarderivate werden unter der Handelsbezeichnung Jaguar^{®} vertrieben und haben die INCI-Bezeichnung Guar Hydroxypropyltrimonium Chloride. Weiterhin werden besonders geeignete kationische Guarderivate auch von der Fa. Hercules unter der Bezeichnung N-Hance^{®} im Handel. Weitere kationische Guarderivate werden von der Fa. Cognis unter der Bezeichnung Cosmedia^{®} vertrieben. Ein bevorzugtes kationisches Guarderivat ist das Handelsprodukt AquaCat^{®} der Fa. Hercules. Bei diesem Rohstoff handelt es sich um ein bereits vorgelöstes kationisches Guarderivat.

Ein weiteres besonders geeignetes kationisches natürliches Polymer stellen Hydrokolloide von Typ der Chitosane dar. Chemisch betrachtet handelt es sich dabei um partiell deacetylierte Chitine unterschiedlichen Molekulargewichtes, die den - idealisierten - Monomerbaustein (I) enthalten:

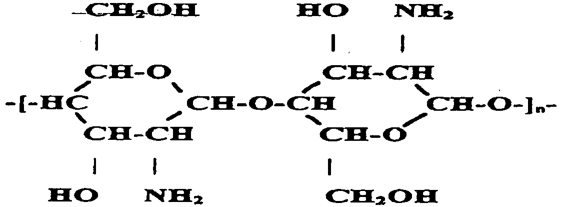

Im Gegensatz zu den meisten Hydrokolloiden, die im Bereich biologischer pH-Werte negativ geladen sind, stellen Chitosane unter diesen Bedingungen kationische Biopolymere dar. Die positiv geladenen Chitosane können mit entgegengesetzt geladenen Oberflächen in Wechselwirkung treten und werden daher in kosmetischen Haar- und Körperpflegemitteln auch als Filmbildner eingesetzt. Neben den Chitosanen kommen auch quaternierte, alkylierte und/oder hydroxyalkylierte Derivate, gegebenenfalls auch in mikrokristalliner Form in Betracht. Der Einsatz kann auch in Form wässriger Gele mit einem Feststoffgehalt im Bereich von 1 bis 5 Gew.-%.

Bei den erfindungsgemäß einzusetzenden Chitosanen handelt es sich um vollständig oder partiell deacetylierte Chitine. Das Molekulargewicht des Chitosans kann über ein breites Spektrum verteilt sein, beispielsweise von 20.000 bis ca. 5 Millionen g/mol. Geeignet ist beispielsweise ein niedermolekulares Chitosan mit einem Molekulargewicht von 30.000 bis 70.000 g/mol. Vorzugsweise liegt das Molekulargewicht jedoch über 100.000 g/mol, besonders bevorzugt von 200. 000 bis 700.000 g/mol. Der Deacetylierungsgrad beträgt vorzugsweise 10 bis 99%, besonders bevorzugt 60 bis 99%.

Die Chitosane oder Chitosanderivate liegen vorzugsweise in neutralisierter oder partiell neutralisierter Form vor. Der Neutralisationsgrad für das Chitosan oder das Chitosanderivat liegt vorzugsweise bei mindestens 50%, besonders bevorzugt zwischen 70 und 100%, bezogen auf die Anzahl der freien Basengruppen. Als Neutralisationsmittel können prinzipiell alle kosmetisch verträglichen anorganischen oder organischen Säuren verwendet werden wie beispielsweise Ameisensäure, Weinsäure, Äpfelsäure, Milchsäure, Zitronensäure, Pyrrolidoncarbonsäure, Salzsäure u.a., von denen die Pyrrolidoncarbonsäure besonders bevorzugt ist.

Ein geeignetes Chitosan wird beispielsweise von der Firma Kyowa Oil& Fat, Japan, unter dem Handelsnamen Flonac^{®} vertrieben. Es hat ein Molekulargewicht von 300.000 bis 700.000 g/mol und ist zu 70 bis 80% entacetyliert. Ein bevorzugtes Chitosansalz ist Chitosoniumpyrrolidoncarboxylat, welches beispielsweise unter der Bezeichnung Kytamer^{®} PC von der Firma Amerchol, USA, vertrieben wird. Das enthaltene Chitosan hat ein Molekulargewicht von ca. 200.000 bis 300.000 g/mol und ist zu 70 bis 85% entacetyliert. Als Chitosanderivate kommen quaternierte, alkylierte oder hydroxyalkylierte Derivate, beispielsweise Hydroxyethyl- oder Hydroxybutylchitosan in Betracht. Weitere Chitosanderivate sind unter den Handelsbezeichnungen Hydagen^{®} CMF, Hydagen^{®} HCMF und Chitolam^{®} NB/101 im Handel frei verfügbar.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden,
- quaternierter Polyvinylalkohol,
- sowie die unter den Bezeichnungen Polyquaternium 2, Polyquaternium 17, Polyquaternium 18 und Polyquaternium 27 bekannten Polymeren mit quartären Stickstoffatomen in der Polymerhauptkette,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind. Weitere in den erfindungsgemäßen Zusammensetzungen einsetzbare kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind.

Erfindungsgemäß bevorzugte kationische Polymere sind kationische Cellulose-Derivate und Chitosan und dessen Derivate, insbesondere die Handelsprodukte Polymer^{®}JR 400, Hydagen^{®} HCMF und Kytamer^{®} PC, kationische Guar-Derivate, kationische Honig-Derivate, insbesondere das Handelsprodukt Honeyquat^{®} 50, kationische Alkylpolyglycodside gemäß der DE-PS 44 13 686 und Polymere vom Typ Polyquaternium-37.

Weiterhin sind kationiserte Proteinhydrolysate zu den kationischen Polymeren zu zählen, wobei das zugrunde liegende Proteinhydrolysat vom Tier, beispielsweise aus Collagen, Milch oder Keratin, von der Pflanze, beispielsweise aus Weizen, Mais, Reis, Kartoffeln, Soja oder Mandeln, von marinen Lebensformen, beispielsweise aus Fischcollagen oder Algen, oder biotechnologisch gewonnenen Proteinhydrolysaten, stammen kann. Die den erfindungsgemäßen kationischen Derivaten zugrunde liegenden Proteinhydrolysate können aus den entsprechenden Proteinen durch eine chemische, insbesondere alkalische oder saure Hydrolyse, durch eine enzymatische Hydrolyse und/oder einer Kombination aus beiden Hydrolysearten gewonnen werden. Die Hydrolyse von Proteinen ergibt in der Regel ein Proteinhydrolysat mit einer Molekulargewichtsverteilung von etwa 100 Dalton bis hin zu mehreren tausend Dalton. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Die Quaternisierung der Proteinhydrolysate oder der Aminosäuren wird häufig mittels quarternären Ammoniumsalzen wie beispielsweise N,N-Dimethyl-N-(n-Alkyl)-N-(2-hydroxy-3-chloro-n-propyl)-ammoniumhalogeniden durchgeführt. Weiterhin können die kationischen Proteinhydrolysate auch noch weiter derivatisiert sein. Als typische Beispiele für die erfindungsgemäßen kationischen Proteinhydrolysate und -derivate seien die unter den INCI - Bezeichnungen im "International Cosmetic Ingredient Dictionary and Handbook", (seventh edition 1997, The Cosmetic, Toiletry, and Fragrance Association 1101 17th Street, N.W., Suite 300, Washington, DC 20036-4702) genannten und im Handel erhältlichen Produkte genannt.

Ganz besonders bevorzugt sind die kationischen Proteinhydrolysate und -derivate auf pflanzlicher Basis.

Die kationischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Bei den anionischen Polymeren (G2) handelt es sich um anionische Polymere, welche Carboxylat- und/oder Sulfonatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Als ganz besonders wirkungsvoll haben sich anionische Polymere erwiesen, die als alleiniges oder Co-Monomer 2-Acrylamido-2-methylpropansulfonsäure enthalten, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfon-säure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 bis 55 Mol-% Acrylamid und 30 bis 45 Mol% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Die anionischen Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Eine weitere ganz besonders bevorzugte Gruppe von Polymeren sind Polyurethane. Die Polyurethane bestehen aus mindestens zwei verschiedenen Monomertypen,
- einer Verbindung (V1) mit mindestens 2 aktiven Wasserstoffatomen pro Molekül und
- einem Di- oder Polyisocyanat (V2).

Bei den Verbindungen (V1) kann es sich beispielsweise um Diole, Triole, Diamine, Triamine, Polyetherole und Polyesterole handeln. Dabei werden die Verbindungen mit mehr als 2 aktiven Wasserstoffatomen üblicherweise nur in geringen Mengen in Kombination mit einem großen Überschuss an Verbindungen mit 2 aktiven Wasserstoffatomen eingesetzt.

Beispiele für Verbindungen (V1) sind Ethylenglykol, 1,2- und 1,3-Propylenglykol, Butylenglykole, Di-, Tri-, Tetra- und Poly-Ethylen- und -Propylenglykole, Copolymere von niederen Alkylenoxiden wie Ethylenoxid, Propylenoxid und Butylenoxid, Ethylendiamin, Propylendiamin, 1,4-Diaminobutan, Hexamethylendiamin und □,□-Diamine auf Basis von langkettigen Alkanen oder Polyalkylenoxiden.

Polyurethane, bei denen die Verbindungen (V1) Diole, Triole und Polyetherole sind, können erfindungsgemäß bevorzugt sein. Insbesondere Polyethylenglykole und Polypropylenglykole mit Molmassen zwischen 200 und 3000, insbesondere zwischen 1600 und 2500, haben sich in einzelnen Fällen als besonders geeignet erwiesen.

Polyesterole werden üblicherweise durch Modifizierung der Verbindung (V1) mit Dicarbonsäuren wie Phthalsäure, Isophthalsäure und Adipinsäure erhalten.

Als Verbindungen (V2) werden überwiegend Hexamethylendiisocyanat, 2,4- und 2,6-Toluoldiisocyanat, 4,4'-Methylendi(phenylisocyanat) und insbesondere Isophorondiisocyanat eingesetzt.

Weiterhin können die erfindungsgemäß verwendeten Polyurethane noch Bausteine wie beispielsweise Diamine als Kettenverlängerer und Hydroxycarbonsäuren enthalten. Dialkylolcarbonsäuren wie beispielsweise Dimethylolpropionsäure sind besonders geeignete Hydroxycarbonsäuren. Hinsichtlich der weiteren Bausteine besteht keine grundsätzliche Beschränkung dahingehend, ob es sich um nichtionische, anionischen oder kationische Bausteine handelt.

Bezüglich weiterer Informationen über den Aufbau und die Herstellung der Polyurethane wird ausdrücklich auf die Artikel in den einschlägigen Übersichtswerken wie Römpps Chemie-Lexikon und Ullmanns Enzyklopädie der technischen Chemie Bezug genommen.

Als in vielen Fälle erfindungsgemäß besonders geeignet haben sich Polyurethane erwiesen, die wie folgt charakterisiert werden können:
- ausschließlich aliphatische Gruppen im Molekül
- keine freien Isocyanatgruppen im Molekül
- Polyether- und Polyesterpolyurethane
- anionische Gruppen im Molekül.

Es hat sich ebenfalls in einigen Fällen als vorteilhaft erwiesen, wenn das Polyurethan in dem System nicht gelöst, sondern stabil dispergiert ist.

Weiterhin hat es sich als für die Herstellung der erfindungsgemäßen Mittel als vorteilhaft erwiesen, wenn die Polyurethane nicht direkt mit den weiteren Komponenten gemischt, sondern in Form von wäßrigen Dispersionen eingebracht wurden. Solche Dispersionen weisen üblicherweise einen Feststoffgehalt von ca. 20-50 %, insbesondere etwa 35-45% auf und sind auch kommerziell erhältlich.

Ein erfindungsgemäß ganz besonders bevorzugtes Polyurethan ist unter der Handelsbezeichnung Luviset^{®} PUR (BASF) im Handel.

Weiterhin können als Polymere amphotere Polymere (G3) verwendet werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻ oder -SO₃-Gruppen enthalten, und solche Polymere zusammengefaßt, die -COOH- oder SO₃H-Gruppen und quartäre Ammoniumgruppen enthalten.

Amphotere Polymere sind ebenso wie die kationischen Polymere ganz besonders bevorzugte Polymere.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Bevorzugt eingesetzte amphotere Polymere sind solche Polymerisate, die sich im wesentlichen zusammensetzen aus
(a) Monomeren mit quartären Ammoniumgruppen der Allgemeinen Formel (G3-I),

   R¹-CH=CR²-CO-Z-(CₙH₂ₙ)-N(⁺⁾R³R⁴R⁵ A⁽⁻⁾ (G3-I)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoffatomen, Z eine NH-Gruppe oder ein Sauerstoffatom, n eine ganze Zahl von 2 bis 5 und A⁽⁻⁾ das Anion einer organischen oder anorganischen Säure ist, und
(b) monomeren Carbonsäuren der Allgemeinen Formel (G3-II),

   R⁶-CH=CR⁷-COOH (G3-II)

   in denen R⁶ und R⁷ unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Ganz besonders bevorzugt sind solche Polymerisate, bei denen Monomere des Typs (a) eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres (a). Als Monomeres (b) für die genannten Polymerisate wird bevorzugt Acrylsäure verwendet.

Geeignete Ausgangsmonomere sind z. B. Dimethylaminoethylacrylamid, Dimethylaminoethylmethacrylamid, Dimethylaminopropylacrylamid, Dimethylaminopropylmethacrylamid und Diethylaminoethylacrylamid, wenn Z eine NH-Gruppe bedeutet oder Dimethylaminoethylacrylat, Dimethylaminoethylmethacrylat und Diethylaminoethylacrylat, wenn Z ein Sauerstoffatom ist.

Die eine tertiäre Aminogruppe enthaltenden Monomeren werden dann in bekannter Weise quarterniert, wobei als Alkylierungsreagenzien Methylchlorid, Dimethylsulfat oder Diethylsulfat besonders geeignet sind. Die Quaternisierungsreaktion kann in wässriger Lösung oder im Lösungsmittel erfolgen.

Vorteilhafterweise werden solche Monomere der Formel (G3-I) verwendet, die Derivate des Acrylamids oder Methacrylamids darstellen. Weiterhin bevorzugt sind solche Monomeren, die als Gegenionen Halogenid-, Methoxysulfat- oder Ethoxysulfat-Ionen enthalten. Ebenfalls bevorzugt sind solche Monomeren der Formel (G3-I), bei denen R³, R⁴ und R⁵ Methylgruppen sind.

Das Acrylamidopropyl-trimethylammoniumchlorid ist ein ganz besonders bevorzugtes Monomer der Formel (G3-I).

Als monomere Carbonsäuren der Formel (G3-II) eignen sich Acrylsäure, Methacrylsäure, Crotonsäure und 2-Methyl-crotonsäure. Bevorzugt werden Acryl- oder Methacrylsäure, insbesondere Acrylsäure, eingesetzt.

Die erfindungsgemäß einsetzbaren zwitterionischen Polymerisate werden aus Monomeren der Formeln (G3-I) und (G3-II) nach an sich bekannten Polymerisationsverfahren hergestellt. Nähere Angaben zum Polymerisationsverfahren können der einschlägigen Fachliteratur entnommen werden.

Als besonders wirksam haben sich solche Polymerisate erwiesen, bei denen die Monomeren der Formel (G3-I) gegenüber den Monomeren der Formel (G3-II) im Überschuss vorlagen. Es ist daher erfindungsgemäß bevorzugt, solche Polymerisate zu verwenden, die aus Monomeren der Formel (G3-I) und die Monomeren der Formel (G3-II) in einem Molverhältnis von 60:40 bis 95:5, insbesondere von 75:25 bis 95:5, bestehen.

Die amphoteren Polymere sind in den erfindungsgemäßen Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weitere erfindungsgemäß einsetzbare amphotere Polymere sind die in der britischen Offenlegungsschrift 2 104 091, der europäischen Offenlegungsschrift 47 714, der europäischen

Offenlegungsschrift 217 274, der europäischen Offenlegungsschrift 283 817 und der deutschen Offenlegungsschrift 28 17 369 genannten Verbindungen. Weiterhin geeignete zwitterionische Polymere sind Methacroylethylbetain/Methacrylat-Copolymere, die unter der Bezeichnung Amersette^{®} (AMERCHOL) im Handel erhältlich sind.

Die erfindungsgemäßen Mittel können in einer weiteren Ausführungsform nichtionogene Polymere (G4) enthalten.

Geeignete nichtionogene Polymere sind beispielsweise:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Celluloseether, wie Hydroxypropylcellulose, Hydroxyethylcellulose und Methylhydroxypropylcellulose, wie sie beispielsweise unter den Warenzeichen Culminal^{®} und Benecel^{®} (AQUALON) und Natrosol^{®}-Typen (Hercules) vertrieben werden.
- Stärke und deren Derivate, insbesondere Stärkeether, beispielsweise Structure^{®} XL (National Starch), eine multifunktionelle, salztolerante Stärke;
- Schellack
- Polyvinylpyrrolidone, wie sie beispielsweise unter der Bezeichnung Luviskol^{®} (BASF) vertrieben werden.
- Siloxane. Diese Siloxane können sowohl wasserlöslich als auch wasserunlöslich sein. Geeignet sind sowohl flüchtige als auch nichtflüchtige Siloxane, wobei als nichtflüchtige Siloxane solche Verbindungen verstanden werden, deren Siedepunkt bei Normaldruck oberhalb von 200 °C liegt. Bevorzugte Siloxane sind Polydialkylsiloxane, wie beispielsweise Polydimethylsiloxan, Polyalkylarylsiloxane, wie beispielsweise Polyphenylmethylsiloxan, ethoxylierte Polydialkylsiloxane sowie Polydialkylsiloxane, die Amin- und/oder HydroxyGruppen enthalten.
- Glycosidisch substituierte Silicone.

Die nichtionischen Polymere sind in den erfindungsgemäßen Zusammensetzungen bevorzugt in Mengen von 0,05 bis 10 Gew.-%, bezogen auf das gesamte Mittel, enthalten. Mengen von 0,1 bis 5 Gew.-% sind besonders bevorzugt.

Polymere können unabhängig von ihrer chemischen Struktur und Ladung auch nach ihrer Funktion in kosmetischen Zusammensetzungen charakterisiert werden. Die Beschreibung der Polymere gemäß ihrer Funktion in den erfindungsgemäßen Zusammensetzungen entspricht nicht zwangsläufig einer Wertung oder Bedeutung dieser Polymere. Vielmehr sind alle Polymere prinzipiell als gleichwertig für die Verwendung in den erfindungsgemäßen Zusammensetzungen anzusehen, wenngleich auch manche dieser Polymere bevorzugt sein können. Weiterhin finden sich manche Polymere aufgrund der Polyfunktionalität von Polymeren in mehreren Beschreibungen bei unterschiedlichen Effekten wieder. Polymere, welche mehrere gewünschte Effekte bewirken können, sind demzufolge besonders bevorzugt bei der Verwendung in den erfindungsgemäßen Zusammensetzungen.

Die Wahl des geeigneten Polymeren richtet sich auch nach der Verwendung der erfindungsgemäßen Zusammensetzung. So wird beispielsweise besonders bevorzugt ein filmbildendes kationisches oder amphoteres Polymer ausgewählt, wenn die Zusammensetzung als Stylingmittel oder Festiger verwendet werden soll.

Da Polymere häufig multifunktional sind, können deren Funktionen nicht immer klar und eindeutig voneinander abgegrenzt werden. Insbesondere gilt dies für filmbildende und festigende Polymere. Dennoch sollen beispielhaft manche filmbildende Polymere beschrieben werden. Allerdings wird an dieser Stelle explizit darauf verwiesen, dass im Rahmen der vorliegenden Erfindung sowohl filmbildende als auch festigende Polymere wesentlich sind. Da beide Eigenschaften auch nicht völlig unabhängig voneinander sind, werden unter dem Begriff "festigende Polymere" auch immer "filmbildende Polymere" verstanden und umgekehrt.

Das filmbildende und/oder festigende Polymer ist in der erfindungsgemäßen Zusammensetzung vorzugsweise in einer Menge von 0,01 bis 40 Gew.%, besonders bevorzugt von 0,1 bis 30 Gewichtsprozent, ganz besonders bevorzugt in einer Menge von 0,1 bis 10 Gewichtsprozent enthalten. Selbstverständlich können auch mehrere filmbildende und/oder festigende Polymere in der erfindungsgemäßen Zusammensetzung enthalten sein. Dabei können diese filmbildenden und/oder festigenden Polymere sowohl permanent als auch temporär kationisch, anionisch, nichtionisch oder amphoter sein. Weiterhin umfasst die vorliegende Erfindung auch die Erkenntnis, dass bei der Verwendung von mindestens zwei filmbildenden und/oder festigenden Polymeren diese selbstverständlich unterschiedliche Ladungen aufweisen können. Erfindungsgemäß bevorzugt kann es sein, wenn ein ionisches filmbildendes und/oder festigendes Polymer mit einem amphoteren und/oder nichtionischem filmbildenen und/oder festigenden Polymer gemeinsam verwendet wird. Auch die Verwendung mindestens zweier gegensätzlich geladener filmbildender und/oder festigender Polymere ist bevorzugt. In letzterem Falle kann eine besondere Ausführungsform wiederum zusätzlich mindestens ein weiteres amphoteres und/oder nichtionisches filmbildendes und/oder festigendes Polymer enthalten.

Die Polymere (G) sind in den erfindungsgemäß verwendeten Zusammensetzungen bevorzugt in Mengen von 0,01 bis 30 Gew.-%, bezogen auf die gesamte Zusammensetzung, enthalten. Mengen von 0,01 bis 25, insbesondere von 0,01 bis 15 Gew.-%, sind besonders bevorzugt.

Mit besonderem Vorzug enthalten die erfindungsgemäßen Zusammensetzungen Fettstoffe (D) als weiteren Wirkstoff. In einer besonders bevorzugen Ausführungsform enthält die erfindungsgemäße Zusammensetzung den erfindungsgemäßen Wirkstoffkomplex aus Arganöl und Sheabutter und einen Fettstoff. Durch diese Zusammensetzung werden erhöhte Mengen der Wirkstoffe auf dem Haar oder der Haut abgeschieden, was zu synergistisch gesteigerten Effekten führt. Dabei wird diese Wirkung durch die weitere Verwendung von kationischen und/oder amphoteren Polymeren als Abscheidungshilfe in den erfindungsgemäßen Zusammensetzungen weiter deutlich erhöht. Dabei erfolgt eine Veränderung der Ladung der Oberfläche, welche beispielsweise durch die Messung der sogenannten Wilhelmy - Spannung gemessen werden kann.

Unter Fettstoffen (D) sind zu verstehen Fettsäuren, Fettalkohole, natürliche und synthetische Wachse, welche sowohl in fester Form als auch flüssig in wässriger Dispersion vorliegen können, und natürliche und synthetische kosmetische Ölkomponenten zu verstehen.

Als Fettsäuren (D1) können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (D2) können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden. Die Fettalkohole werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern (D4) sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ -Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkoholcaprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-diisotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,
- Fettsäurepartialglyceride, das sind Monoglyceride, Diglyceride und deren technische Gemische. Bei der Verwendung technischer Produkte können herstellungsbedingt noch geringe Mengen Triglyceride enthalten sein. Die Partialglyceride folgen vorzugsweise der Formel (D4-I), 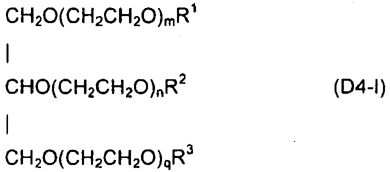 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Bevorzugt steht R¹ für einen Acylrest und R² und R³ für Wasserstoff und die Summe (m+n+q) ist 0. Typische Beispiele sind Mono- und/oder Diglyceride auf Basis von Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Ölsäuremonoglyceride eingesetzt.

Die Einsatzmenge der natürlichen und synthetischen kosmetischen C5lkörper in den erfindungsgemäß verwendeten Mitteln beträgt üblicherweise 0,1 - 30 Gew.%, bezogen auf das gesamte Mittel, bevorzugt 0,1 - 20 Gew.-%, und insbesondere 0,1 - 15 Gew.-%.

Eine letzte Substanzgruppe, welche als Fettstoffe verwendet werden können, sind Silikonöle.

Silikonöle (S) bewirken die unterschiedlichsten Effekte. So beeinflussen sie beispielsweise gleichzeitig die Trocken- und Nasskämmbarkeiten, den Griff des trockenen und nassen Haares sowie den Glanz. Aber auch die Weichheit und die Elastizität des Filmes, welcher durch filmbildende Polymere auf dem Haar zum Zwecke der Festigung und des Stylens gebildet wird, wird durch Silikone positiv beeinflusst. Unter dem Begriff Silikonöle versteht der Fachmann mehrere Strukturen Siliciumorganischer Verbindungen. Zunächst werden hierunter die Dimethiconole (S1) verstanden. Dimethiconole bilden die erste Gruppe der Silikone, welche erfindungsgemäß besonders bevorzugt sind. Die erfindungsgemäßen Dimethiconole können sowohl linear als auch verzweigt als auch cyclisch oder cyclisch und verzweigt sein. Lineare Dimethiconole können durch die folgende Strukturformel (S1 - I) dargestellt werden:

(SiOHR¹₂) - O - (SiR²₂ - O - )ₓ - (SiOHR¹₂) (S1 - I)

Verzweigte Dimethiconole können durch die Strukturformel (S1 - II) dargestellt werden:

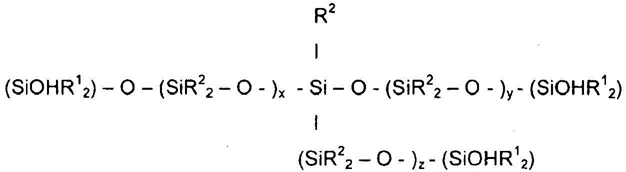

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-,-(CH₂)₃ CC(O)OCH₂CH₂-, -C₆H₄C₆H₄-, -C₆H₄CH₂C₆H₄-; und -(CH₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Vsikositäten liegen zwischen 10000 und 3000000 cPs. Der bevorzugteste Bereich liegt zwischen 50000 und 2000000 cPs. Selbstverständlich umfasst die erfindungsgemäße Lehre auch, dass die Dimethiconole bereits als Emulsion vorliegen können. Dabei kann die entsprechende Emulsion der Dimethiconole sowohl nach der Herstellung der entsprechenden Dimethiconole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconole als Emulsion verwendet werden, dann beträgt die Tröpfchengröße der emulgierten Teilchen erfindungsgemäß 0,01µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethiconole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconole ganz besonders bevorzugt sein.

Als Beispiele für derartige Produkte werden die folgenden Handelsprodukte genannt: Botanisil NU-150M (Botanigenics), Dow Corning 1-1254 Fluid, Dow Corning 2-9023 Fluid, Dow Corning 2-9026 Fluid, Ultrapure Dimethiconol (Ultra Chemical), Unisil SF-R (Universal Preserve), X-21-5619 (Shin-Etsu Chemical Co.), Abil OSW 5 (Degussa Care Specialties), ACC DL-9430 Emulsion (Taylor Chemical Company), AEC Dimethiconol & Sodium Dodecylbenzenesulfonate (A & E Connock (Perfumery & Cosmetics) Ltd.), B C Dimethiconol Emulsion 95 (Basildon Chemical Company, Ltd.), Cosmetic Fluid 1401, Cosmetic Fluid 1403, Cosmetic Fluid 1501, Cosmetic Fluid 1401DC (alle zuvor genannten Chemsil Silicones, Inc.), Dow Corning 1401 Fluid, Dow Corning 1403 Fluid, Dow Corning 1501 Fluid, Dow Corning 1784 HVF Emulsion, Dow Corning 9546 Silicone Elastomer Blend (alle zuvor genannten Dow Corning Corporation), Dub Gel SI 1400 (Stearinerie Dubois Fils), HVM 4852 Emulsion (Crompton Corporation), Jeesilc 6056 (Jeen International Corporation), Lubrasil, Lubrasil DS ( beide Guardian Laboratories), Nonychosine E, Nonychosine V (beide Exsymol), SanSurf Petrolatum-25, Satin Finish ( beide Collaborative Laboratories, Inc.), Silatex-D30 (Cosmetic Ingredient Resources), Silsoft 148, Silsoft E-50, Silsoft E-623 (alle zuvor genannten Crompton Corporation), SM555, SM2725, SM2765, SM2785 (alle zuvor genannten GE Silicones), Taylor T-Sil CD-1, Taylor TME-4050E (alle Taylor Chemical Company), TH V 148 (Crompton Corporation), Tixogel CYD-1429 (Sud-Chemie Performance Additives), Wacker-Belsil CM 1000, Wacker-Belsil CM 3092, Wacker-Belsil CM 5040, Wacker-Belsil DM 3096, Wacker-Beisil DM 3112 VP, Wacker-Belsil DM 8005 VP, Wacker-Belsil DM 60081 VP (alle zuvor genannten Wacker-Chemie GmbH).

Die Dimethiconole (S1) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconol bezogen auf die Zusammensetzung.

Erfindungsgemäß ist es auch möglich, dass die Dimethiconole eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Dimethiconol bis zu 40 Gew.%, bevorzugt in Mengen von bis zu 25 Gew.%bezogen auf die Gesamtzusammensetzung betragen.

Dimethicone (S2) bilden die zweite Gruppe der Silikone, welche erfindungsgemäß besonders bevorzugt sind. Die erfindungsgemäßen Dimethicone können sowohl linear als auch verzweigt als auch cyclisch oder cyclisch und verzweigt sein. Lineare Dimethicone können durch die folgende Strukturformel (S2 - I) dargestellt werden:

(SIR¹₃) - O - (SiR²₂ - O-)ₓ-(SiR¹₃) (S2 - I)

Verzweigte Dimethicone können durch die Strukturformel (S2 - II) dargestellt werden:

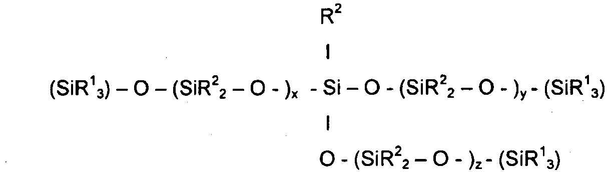

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-,-(CH₂)₃ CC(O)OCH₂CH₂-, -C₆H₄C₆H₄-, -C₆H₄CH₂C₆H₄-; und -(CH₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, banz besonders bevorzugte Vsikositäten liegen zwischen 10000 und 3000000 cPs. Der bevorzugteste Bereich liegt zwischen 50000 und 2000000 cPs. Selbstverständlich umfasst die erfindungsgemäße Lehre auch, dass die Dimethicone bereits als Emulsion vorliegen können. Dabei kann die entsprechende Emulsion der Dimethicone sowohl nach der Herstellung der entsprechenden Dimethicone aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethicone auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethicone als Emulsion verwendet werden, dann beträgt die Tröpfchengröße der emulgierten Teilchen erfindungsgemäß 0,01 µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethicone verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethicone ganz besonders bevorzugt sein.

Die Dimethicone (S2) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconon bezogen auf die Zusammensetzung.

Erfindungsgemäß ist es auch möglich, dass die Dimethicone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Dimethicon bis zu 40 Gew.%, bevorzugt in Mengen von bis zu 25 Gew.% bezogen auf die Gesamtzusammensetzung betragen.

Dimethiconcopolyole (S3) bilden eine weitere Gruppe bevorzugter Silikone. Dimethiconcopolyole können durch die folgende Strukturformeln dargestellt werden:

(SiR¹₃)-O-(SiR²₂-O-)ₓ- (SiRPE - O-)_{y}-(SiR¹₃) (S3 - I)

oder durch die nachfolgende Strukturformel:

PE - (SiR¹₂) - O - (SiR²₂ - O-)ₓ-(SiR¹₂)- PE (S3 - II)

Verzweigte Dimethiconcopolyole können durch die Strukturformel (S3 - III) dargestellt werden: 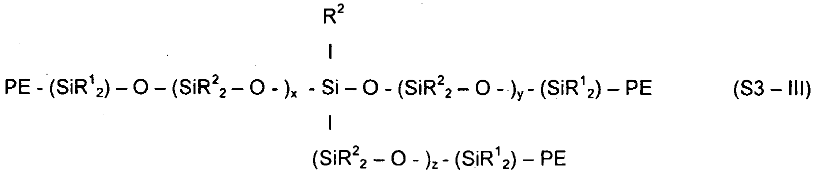 oder durch die Strukturformel(S3 - IV):

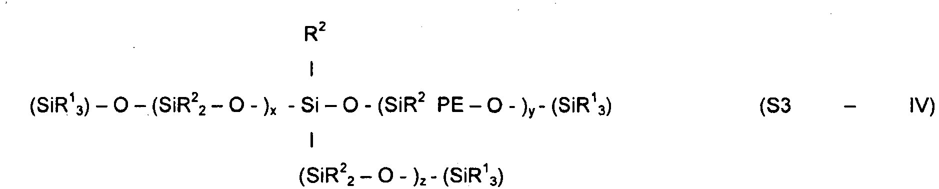

Die Reste R¹ und R² stehen unabhängig voneinander jeweils für Wasserstoff, einen Methylrest, einen C2 bis C30 linearen, gesättigten oder ungesättigten Kohlenwasserstoffrest, einen Phenylrest und/oder eine Arylrest. Nicht einschränkende Beispiele der durch R¹ und R² repräsentierten Reste schließen Alkylreste, wie Methyl, Ethyl, Propyl, Isopropyl, Butyl, Isobutyl, Pentyl, Isopentyl, Neopentyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R¹ und R² ein Alkylrest, der 1 bis etwa 6 Kohlenstoffatomen enthält, und am bevorzugtesten ist R¹ und R² Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-,-(CH₂)₃ CC(O)OCH₂CH₂-, -C₆H₄C₆H₄-, -C₆H₄CH₂C₆H₄-; und -(CH₂)₃C(O)SCH₂CH₂- ein. Bevorzugt als R¹ und R² sind Methyl, Phenyl und C2 bis C22 - Alkylreste. Bei den C2 bis C22 Alkylresten sind ganz besonders Lauryl-, Stearyl-, und Behenylreste bevorzugt. PE steht für einen Polyoxyalkylenrest. Bevorzugte Polyoxyalkylenreste leiten sich ab von Ethylenoxid, Propylenoxid und Glycerin. Die Zahlen x, y und z sind ganze Zahlen und laufen jeweils unabhängig voneinander von 0 bis 50.000. Die Molgewichte der Dimethicone liegen zwischen 1000 D und 10000000 D. Die Viskositäten liegen zwischen 100 und 10000000 cPs gemessen bei 25 °C mit Hilfe eines Glaskapillarviskosimeters nach der Dow Corning Corporate Testmethode CTM 0004 vom 20. Juli 1970. Bevorzugte Viskositäten liegen zwischen 1000 und 5000000 cPs, ganz besonders bevorzugte Viskositäten liegen zwischen 10000 und 3000000 cPs. Der bevorzugteste Bereich liegt zwischen 50000 und 2000000 cPs.

Selbstverständlich umfasst die erfindungsgemäße Lehre auch, dass die Dimethiconcopolymere bereits als Emulsion vorliegen können. Dabei kann die entsprechende Emulsion der Dimethiconcopolyole sowohl nach der Herstellung der entsprechenden Dimethiconcopolyole aus diesen und den dem Fachmann bekannten üblichen Verfahren zur Emulgierung hergestellt werden. Hierzu können als Hilfsmittel zur Herstellung der entsprechenden Emulsionen sowohl kationische, anionische, nichtionische oder zwitterionische Tenside und Emulgatoren als Hilfsstoffe verwendet werden. Selbstverständlich können die Emulsionen der Dimethiconcopolyole auch direkt durch ein Emulsionspolymerisationsverfahren hergestellt werden. Auch derartige Verfahren sind dem Fachmann wohl bekannt. Hierzu sei beispielsweise verwiesen auf die "Encyclopedia of Polymer Science and Engineering, Volume 15, Second Edition, Seiten 204 bis 308, John Wiley & Sons, Inc. 1989. Auf dieses Standardwerk wird ausdrücklich Bezug genommen.

Wenn die erfindungsgemäßen Dimethiconcopolyole als Emulsion verwendet werden, dann beträgt die Tröpfchengröße der emulgierten Teilchen erfindungsgemäß 0,01 µm bis 10000 µm, bevorzugt 0,01 bis 100 µm, ganz besonders bevorzugt 0,01 bis 20 µm und am bevorzugtesten 0,01 bis 10 µm. Die Teilchengröße wird dabei nach der Methode der Lichtstreuung bestimmt.

Werden verzweigte Dimethiconcopolyole verwendet, so ist darunter zu verstehen, dass die Verzweigung größer ist, als eine zufällige Verzweigung, welche durch Verunreinigungen der jeweiligen Monomere zufällig entsteht. Im Sinne der vorliegenden Verbindung ist daher unter verzweigten Dimethiconcopolyolen zu verstehen, dass der Verzweigungsgrad größer als 0,01 % ist. Bevorzugt ist ein Verzweigungsgrad größer als 0,1 % und ganz besonders bevorzugt von größer als 0,5 %. Der Grad der Verzweigung wird dabei aus dem Verhältnis der unverzweigten Monomeren, das heißt der Menge des monofunktionalen Siloxanes, zu den verzweigenden Monomeren, das heißt der Menge an tri- und tetrafunktionalen Siloxanen, bestimmt. Erfindungsgemäß können sowohl niedrigverzweigte als auch hochverzweigte Dimethiconcopolyole ganz besonders bevorzugt sein.

Die Dimethiconcopolyole (S3) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Dimethiconcopolyol bezogen auf die Zusammensetzung. Erfindungsgemäß ist es auch möglich, dass die Dimethiconcopolyole eine eigene Phase in den erfindungsgemäßen Zusammensetzungen bilden. In diesem Falle kann die Menge an Dimethiconcopolyol bis zu 40 Gew.%, bevorzugt in Mengen von bis zu 25 Gew.% bezogen auf die Gesamtzusammensetzung betragen.

Aminofunktionelle Silikone oder auch Amodimethicone (S4) genannt, sind Silicone, welche mindestens eine (gegebenenfalls substituierte) Aminogruppe aufweisen.
Solche Silicone können z.B. durch die Formel (S4 -I)

M(RₐQ_{b}SiO_{(4-a-b)/2)x}(R_{c}SiO_{(4-c)/2)y}M (S4 - I)

beschreiben werden, wobei in der obigen Formel R ein Kohlenwasserstoff oder ein Kohlenwasserstoffrest mit 1 bis etwa 6 Kohlenstoffatomen ist, Q ein polarer Rest der Allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stande der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-,-CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H₄C₆H₄-, -C₆H₄CH₂C₆H₄-; und -(CH₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂)_{z}NH₂, worin z 1 oder mehr ist. Eine andere mögliche Formel für Z ist -NH(CH₂)_{z}(CH₂)_{zz}-NH, worin sowohl z als auch zz unabhängig 1 oder mehr sind, wobei diese Struktur Diamino-Ringstrukturen umfaßt, wie Piperazinyl. Z ist am bevorzugtesten ein -NHCH₂CH₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b}SiO_{(4-a-b})_{/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Bevorzugte erfindungsgemäße Mittel sind dadurch gekennzeichnet, dasa sie ein aminofunktionelles Silikon der Formel (S4 - II)

R'ₐG₃₋ₐ-Si(OSiG₂)ₙ-(OSiG_{b}R'_{2-b})ₘ-O-SiG₃₋ₐ-R'ₐ (S4 - II),

enthalten, worin bedeutet:
- G ist-H, eine Phenylgruppe, -OH, -O-CH₃, -CH₃, -CH₂CH₃, -CH₂CH₂CH₃, -CH(CH₃)₂, -CH₂CH₂CH₂H₃, -CH₂CH(CH₃)₂, -CH(CH₃)CH₂CH₃, -C(CH₃)₃;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -N(R")-CH₂-CH₂- N(R")₂
   ∘ -N(R")₂
   ∘ -N⁺(R")₃A⁻
   ∘ -N⁺H(R")₂ A⁻
   ∘ -N⁺H₂(R")A⁻
   ∘ -N(R")-CH₂-CH₂-N⁺R"H₂A⁻,
      wobei jedes R" für gleiche oder verschiedene Reste aus der Gruppe -H, - Phenyl, -Benzyl, der C₁₋₂₀-Alkylreste, vorzugsweise -CH₃, -CH₂CH₃,-CH₂CH₂CH₃, -CH(CH₃)₂, -CH₂CH₂CH₂H₃, -CH₂CH(CH₃)₂, -CH(CH₃)CH₂CH₃,-C(CH₃)₃, steht und A ein Anion repräsentiert, welches vorzugsweise ausgewählt ist aus Chlorid, Bromid, Iodid oder Methosulfat.

Besonders bevorzugte erfindungsgemäße Mittel sind dadurch gekennzeichnet, dasa sie ein aminofunktionelles Silikon der Formel (S4 - III) 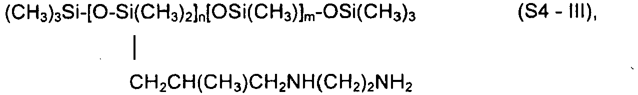 enthalten, worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt. Diese Silicone werden nach der INCI-Deklaration als Trimethylsilylamodimethicone bezeichnet. Besonders bevorzugt sind auch erfindungsgemäße Mittel, die dadurch gekennzeichnet sind, dasa sie ein aminofunktionelles Silikon der Formel (S4 - IV) 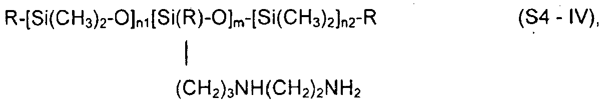 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.
Diese Silicone werden nach der INCI-Deklaration als Amodimethicone bezeichnet.

Unabhängig davon, welche aminofunktionellen Silicone eingesetzt werden, sind erfindungsgemäße Mittel bevorzugt, bei denen das aminofunktionelle Silikon eine Aminzahl oberhalb von 0,25 meq/g, vorzugsweise oberhalb von 0,3 meq/g und insbesondere oberhalb von 0,4 meq/g aufweist. Die Aminzahl steht dabei für die Milli-Äquivalente Amin pro Gramm des aminofunktioinelen Silicons. Sie kann durch Titration ermittelt und auch in der Einheit mg KOH/g angegeben werden.

Die Amodimethicone (S4) sind in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Amodimethicon bezogen auf die Zusammensetzung.

Erfindungsgemäß ist es auch möglich, dass die Amodimethicone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Amodimethicon bis zu 40 Gew.%, bevorzugt in Mengen von bis zu 25 Gew.% bezogen auf die Gesamtzusammensetzung betragen.

Erst seit kurzem sind völlig neuartige Polyammmonium-Polysiloxan Verbindungen bekannt, in welchen die Siloxansubstrukturen gegebenenfalls über Ammoniumsubstrukturen miteinander verbunden sind. Derartige Verbindungen und deren Verwendung in kosmetischen Mitteln werden beispielsweise in der Offenlegungsschrift WO 02/10257 beschrieben.

Die vorstehend beschriebenen Polyammonium-Polysiloxan Verbindungen können beispielsweise unter der Handelsbezeichnung Baysilone^{®} von GE Bayer Silicones bezogen werden. Die Produkte mit den Bezeichnungen Baysilone TP 3911, SME 253 und SFE 839 sind dabei bevorzugt. Ganz besonders bevorzugt ist die Verwendung von Baysilone TP 3911 als Wirkkomponente der erfindungsgemäßen Zusammensetzungen.

Die vorstehend beschriebenen Polyammonium-Polysiloxan Verbindungen werden in dem erfindungsgemäßen Zusammensetzungen in einer Menge von 0,01 bis 10 Gew.% , vorzugsweise 0,01 bis 7,5, besonders bevorzugt 0,01 bis 5,0 Gew.%, ganz besonders bevorzugt von 0,05 bis 2,5 Gew.% jeweils in Bezug auf die Gesamtzusammensetzung verwendet.

Das Verhältnis der Polyammonium-Polysiloxan Verbindungen zu einer weiteren synergistischen Wirkstoffkomponente ausgewählt aus der Gruppe der Polymere, der Proteinhydrolysate, der Silikone, der Vitamine und der Pflanzenextrakte beträgt im Allgemeinen erfindungsgemäß 1:1000 bis 1:2, vorzugsweise 1:100 bis 1:2, besonders bevorzugt 1:50 bis 1:2 und ganz besonders bevorzugt 1:10 bis 1:2.

Wenn eine Mischung aus mindestens zwei Silikonen verwendet wird, so ist diese Mischung in den erfindungsgemäßen Zusammensetzungen in Mengen von 0,01 bis 10 Gew.%, vorzugsweise 0,01 bis 8 Gew.%, besonders bevorzugt 0,1 bis 7,5 Gew.% und insbesondere 0,1 bis 5 Gew.% an Silikonmischung bezogen auf die Zusammensetzung enthalten.

Erfindungsgemäß ist es auch möglich, dass die Mischung der Silikone eine eigene Phase in den erfindungsgemäßen Zusammensetzungen bilden. In diesem Fall kann es angebracht sein, wenn die Zusammensetzung unmittelbar vor der Anwendung durch Schütteln kurzfristig homogenisiert wird. In diesem Falle kann die Menge an Silikonmischung bis zu 40 Gew.%, bevorzugt in Mengen von bis zu 25 Gew.%bezogen auf die Gesamtzusammensetzung betragen.

Selbstverständlich umfasst die erfindungsgemäße Lehre auch, dass eine Mischung aus mehreren Fettstoffen (D) aus unterschiedlichen Klassen von Fettstoffen, mindestens zwei unterschiedlichen Fettstoffklassen in den erfindungsgemäßen Zusammensetzungen verwendet werden kann. Die bevorzugten Mischungen aus mindestens zwei Öl- und Fettkomponenten enthalten in diesem Falle zwingend mindestens eine weitere Silikonkomponente. Bevorzugt wird die Silikonkomponente in diesem Falle ausgewählt aus den Dimethiconolen und den Amodimethiconen.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Ein weiterer erfindungsgemäßer synergistischer Wirkstoff in den erfindungsgemäßen Zusammensetzungen mit dem erfindungsgemäßen Wirkstoffkomplex sind Proteinhydrolysate und/oder dessen Derivate (P).

Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Weiterhin werden erfindungsgemäß aus Aminosäuren und Aminosäurederivaten aufgebaute Polymere unter dem Begriff Proteinhydrolysate verstanden. Zu letzteren sind beispielsweise Polyalanin, Polyasparagin, Polyserin etc. zu zählen. Weitere Beispiele für erfindungsgemäß einsetzbare Verbindungen sind L-Alanyl-L-prolin, Polyglycin, Glycyl-L-glutamin oder D/L-Methionin-S-Methylsulfoniumchlorid. Selbstverständlich können erfindungsgemäß auch β-Aminosäuren und deren Derivate wie ß-Alanin, Anthranilsäure oder Hippursäure eingesetzt werden. Das Molgewicht der erfindungsgemäß einsetzbaren Proteinhydrolysate liegt zwischen 75, dem Molgewicht für Glycin, und 200000, bevorzugt beträgt das Molgewicht 75 bis 50000 und ganz besonders bevorzugt 75 bis 20000 Dalton. Selbstverständlich umfasst die vorliegende erfindungsgemäße Lehre auch, dass im Falle der Aminosäuren diese in Form von Derivaten, wie beispielsweise der N-Acylderivate, der N-Alkyl oder der O-Ester vorliegen können. Im Falle der N-acylderivate ist die Acylgruppe eine Formylrest, ein Acetylrest, ein Propionylrest, ein Butyrylrest oder der Rest einer geradkettigen, verzweigten oder unverzweigten, gesättigten oder ungesättigten Fettsäure mit einer Kettenlänge von 8 bis 30 C-Atomen. Im Falle einer N-Alkylderivate kann die Alkylgruppe linear, verzweigt, gesättigt oder ungesättigt sein und hat eine C-Kettenlänge von 1 bis 30 C-Atomen. Im Falle der O-Ester sind die der Veresterung zugrunde liegenden Alkohole Methanol, Ethanol, Isopropanol, Propanol, Butanol, Isobutanol, Pentanol, Neopentanol, Isopentanol, Hexanole, Heptanole, Capryl- oder Capronalkohol, Octanole, Nonanole, Decanole, Dodecanole, Lauranole, insbesondere gesättigte oder ungesättigte, lineare oder verzweigte Alkohole mit einer C-Kettenlänge von 1 bis 30 C-Atomen. Selbstverständlich können die Aminosäuren sowohl am N-Atom als auch am O-Atom gleichzeitig derivatisiert sein. Selbstverständlich können die Aminosäuren auch in Salzform, insbesondere als Mischsalze zusammen mit Genusssäuren verwendet werden. Dies kann erfindungsgemäß bevorzugt sein.

Als Beispiele für Aminosäuren und deren Derivaten als erfindungsgemäße Proteinhydrolysate werden genannt: Alanin, Arginin, Carnitin, Creatin, Cystathionin, Cystein, Cystin, Cystinsäure, Glycin, Histidin, Homocystein, Homoserin, Isoleucin, Lanthionin, Leucin, Lysin, Methionin, Norleucin, Norvalin, Ornithin, Phenylalanin, Prolin, Hydroxyprolin, Sarcosin, Serin, Threonin, Tryptophan, Thyronin, Tyrosin, Valin, Asparaginsäure, Asparagin, Glutaminsäure und Glutamin. Bevorzugte Aminosäuren sind Alanin, Arginin, Glycin, Histidin, Lanthionin, Leucin, Lysin, Prolin, Hydroxyprolin Serin und Asparagin. Ganz besonders bevorzugt werden verwendet Alanin, Glycin, Histidin, Lysin, Serin und Arginin. Am bevorzugtesten werden Glycin, Histidin, Lysin und Serin verwendet.

Erfindungsgemäß können Proteinhydrolysate sowohl pflanzlichen als auch tierischen oder marinen oder synthetischen Ursprungs eingesetzt werden.

Tierische Proteinhydrolysate sind beispielsweise Elastin-, Kollagen-, Keratin-, Seiden- und Milcheiweiß-Proteinhydrolysate, die auch in Form von Salzen vorliegen können. Solche Produkte werden beispielsweise unter den Warenzeichen Dehylan^{®} (Cognis), Promois^{®} (Interorgana), Collapuron^{®} (Cognis), Nutrilan^{®} (Cognis), Gelita-Sol^{®} (Deutsche Gelatine Fabriken Stoess & Co), Lexein^{®} (Inolex) und Kerasol^{®} (Croda) vertrieben.

Weiterhin sind erfindungsgemäß bevorzugte pflanzliche Proteinhydrolysaten wie beispielsweise Soja-, Mandel-, Erbsen-, Kartoffel- und Weizenproteinhydrolysate. Solche Produkte sind beispielsweise unter den Warenzeichen Gluadin^{®} (Cognis), DiaMin^{®} (Diamalt), Lexein^{®} (Inolex), Hydrosoy^{®} (Croda), Hydrolupin^{®} (Croda), Hydrosesame^{®} (Croda), Hydrotritium^{®} (Croda) und Crotein^{®} (Croda) erhältlich.

Weitere erfindungsgemäß bevorzugte Proteinhydrolysate sind maritimen Ursprunges. Hierzu zählen beispielsweise Kollagenhydrolysate von Fischen oder Algen sowie Proteinhydrolysate von Muscheln bzw. Perlenhydrolysate.

Beispiele für erfindungsgemäße Perlenextrakte sind die Handelsprodukte Pearl Protein Extract BG^{®} oder Crodarom^{®} Pearl.

In den kosmetischen Zusammensetzungen ist einer der zuvor beschriebenen Perlenextrakte in einer Menge von mindestens 0,01 bis zu 20 Gew.% enthalten. Bevorzugt werden Mengen des Extraktes von 0,01 bis zu 10 Gew.%, ganz besonders bevorzugt Mengen von 0,01 bis 5 Gew.% bezogen auf die gesamte kosmetische Zusammensetzung verwendet.

Ein weiteres ganz besonderes Proteinhydrolysat wird aus der Seide gewonnen.

In den erfindungsgemäß verwendeten Mitteln sind die Seidenproteinhydroysate und/oder deren Derivate in Mengen von 0,001 - 10 Gew.-% bezogen auf das gesamte Mittel enthalten. Mengen von 0,005 bis 5, insbesondere 0,01 bis 3 Gew.-%, sind ganz besonders bevorzugt.

Wenngleich der Einsatz der Proteinhydrolysate als solche bevorzugt ist, können an deren Stelle gegebenenfalls auch anderweitig erhaltene Aminosäuregemische eingesetzt werden. Ebenfalls möglich ist der Einsatz von Derivaten der Proteinhydrolysate, beispielsweise in Form ihrer Fettsäure-Kondensationsprodukte. Solche Produkte werden beispielsweise unter den Bezeichnungen Lamepon^{®} (Cognis), Lexein^{®} (Inolex), Crolastin^{®} (Croda) oder Crotein^{®} (Croda) vertrieben.

Selbstverständlich umfaßt die erfindungsgemäße Lehre alle isomeren Formen, wie cis - trans - Isomere, Diastereomere und chirale Isomere.

Erfindungsgemäß ist es auch möglich, eine Mischung aus mehreren Proteinhydrolysaten (P) einzusetzen.

Die Proteinhydrolysate (P) sind in den Zusammensetzungen in Konzentrationen von 0,001 Gew.% bis zu 20 Gew.%, vorzugsweise von 0,05 Gew.% bis zu 15 Gew.% und ganz besonders bevorzugt in Mengen von 0,05 Gew.% bis zu 5 Gew.% enthalten.

Die Wirkung der erfindungsgemäßen Zusammensetzungen kann weiterhin durch eine 2-Pyrrolidinon-5-carbonsäure und deren Derivate (J) gesteigert werden. Ein weiterer Gegenstand der Erfindung ist daher die Verwendung von Derivaten der 2-Pyrrolidinon-5-carbonsäure. Bevorzugt sind die Natrium-, Kalium-, Calcium-, Magnesium- oder Ammoniumsalze, bei denen das Ammoniumion neben Wasserstoff eine bis drei C₁- bis C₄-Alkylgruppen trägt. Das Natriumsalz ist ganz besonders bevorzugt. Die eingesetzten Mengen in den erfindungsgemäßen Mitteln betragen 0,05 bis 10 Gew.%, bezogen auf das gesamte Mittel, besonders bevorzugt 0,1 bis 5, und insbesondere 0,1 bis 3 Gew.%.

Eine weitere bevorzugte Gruppe von Inhaltsstoffen der erfindungsgemäßen Zusammensetzungen mit dem erfindungsgemäßen Wirkstoffkomplex sind Vitamine, Provitamine oder Vitaminvorstufen. Vitamine, Pro-Vitamine und Vitaminvorstufen sind dabei besonders bevorzugt, die den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht. Die erfindungsgemäßen Mittel enthalten die Vitamin A-Komponente bevorzugt in Mengen von 0,05-1 Gew.-%, bezogen auf die gesamte Zubereitung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das in den erfindungsgemäß verwendeten Mitteln bevorzugt in Mengen von 0,05 bis 1 Gew.-%, bezogen auf das gesamte Mittel, enthalten ist.
- Vitamin B₅ (Pantothensäure, Panthenol und Pantolacton). Im Rahmen dieser Gruppe wird bevorzugt das Panthenol und/oder Pantolacton eingesetzt. Erfindungsgemäß einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie die in der WO 92/13829 offenbarten kationischen Panthenolderivate. Die genannten Verbindungen des Vitamin B₅-Typs sind in den erfindungsgemäßen Mitteln bevorzugt in Mengen von 0,05 - 10 Gew.-%, bezogen auf das gesamte Mittel, enthalten. Mengen von 0,1 - 5 Gew.-% sind besonders bevorzugt.
- Vitamin B₆ (Pyridoxin sowie Pyridoxamin und Pyridoxal).

Vitamin C (Ascorbinsäure). Vitamin C wird in den erfindungsgemäßen Mitteln bevorzugt in Mengen von 0,1 bis 3 Gew.-%, bezogen auf das gesamte Mittel eingesetzt. Die Verwendung in Form des Palmitinsäureesters, der Glucoside oder Phosphate kann bevorzugt sein. Die Verwendung in Kombination mit Tocopherolen kann ebenfalls bevorzugt sein.

Vitamin E (Tocopherole, insbesondere α-Tocopherol). Tocopherol und seine Derivate, worunter insbesondere die Ester wie das Acetat, das Nicotinat, das Phosphat und das Succinat fallen, sind in den erfindungsgemäßen Mitteln bevorzugt in Mengen von 0,05-1 Gew.-%, bezogen auf das gesamte Mittel, enthalten.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3aS,4S, 6aR)-2-Oxohexahydrothienol[3,4-d]-imidazol-4-valeriansäure bezeichnet, für die sich aber inzwischen der Trivialname Biotin durchgesetzt hat. Biotin ist in den erfindungsgemäßen Mitteln bevorzugt in Mengen von 0,0001 bis 1,0 Gew.-%, insbesondere in Mengen von 0,001 bis 0,01 Gew.-% enthalten.

Bevorzugt enthalten die erfindungsgemäßen Zusammensetzungen Vitamine, Provitamine und Vitaminvorstufen aus den Gruppen A, B, E und H. Panthenol, Pantolacton, Pyridoxin und seine Derivate sowie Nicotinsäureamid und Biotin sind besonders bevorzugt.

Schließlich ergeben sich durch die Verwendung von Pflanzenextrakten (L) in den erfindungsgemäßen Zusammensetzungen weitere synergistische Vorteile. Daher ist die Verwendung dieser Substanzen besonders vorteilhaft.

Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind.

Die Pflanzenextrakte können erfindungsgemäß sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Weiterhin kann es bevorzugt sein, in den erfindungsgemäßen Mitteln Mischungen aus mehreren, insbesondere aus zwei, verschiedenen Pflanzenextrakten einzusetzen.

Zusätzlich kann es sich als vorteilhaft erweisen, wenn in den erfindungsgemäßen Zusammensetzungen Penetrationshilfsstoffe und/ oder Quellmittel (M) enthalten sind. Diese Hilfsstoffe sorgen für eine bessere Penetration von Wirkstoffen in die keratinische Faser oder helfen die keratinische Faser aufzuquellen. Hierzu sind beispielsweise zu zählen Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Der pH-Wert der erfindungsgemäßen Zubereitungen kann prinzipiell bei Werten von 2 - 11 liegen. Der pH - Wert wird je nach dem Zweck und der Verwendung der erfindungsgemäßen Zusammensetzung ganz gezielt ausgewählt und eingestellt. Für Färbemittel liegt er beispielsweise bevorzugt zwischen 5 und 11, wobei Werte von 6 bis 10 besonders bevorzugt sind. Für reinigende Zusammensetzungen liegt er beispielsweise zwischen 4 und 7,5, bevorzugt zwischen 4 und 6.

Zur Einstellung dieses pH-Wertes kann praktisch jede für kosmetische Zwecke verwendbare Säure oder Base verwendet werden. Bevorzugte Basen sind Ammoniak, Alkalihydroxide, Monoethanolamin, Triethanolamin sowie N,N,N',N'-Tetrakis-(2-hydroxypropyl)-ethylendiamin. Üblicherweise werden als Säuren Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Es wurde weiterhin gefunden, dasa die Wirkung des erfindungsgemäßen Wirkstoffes in den erfindungsgemäßen Mitteln in Kombination mit Stoffen, welche primäre oder sekundäre Aminogruppen enthalten, weiter gesteigert werden kann. Als Beispiele für derartige Aminoverbindungen seien genannt Ammoniak, Monoethanolamin, 2-Amino-2-methyl-1-propanol, 2-Amino-2-methyl-propandiol sowie basische Aminosäuren wie beispielsweise Lysin, Arginin oder Histidin. Selbstverständlich können diese Amine auch in Form der entsprechenden Salze mit anorganischen und/oder organischen Säuren eingesetzt werden, wie beispielsweise als Ammoniumcarbonat, Ammoniumcitrat, Ammoniumoxalat, Ammoniumtartrat oder Lysinhydrochlorid. Die Amine werden mit dem erfindungsgemäßen Wirkstoff gemeinsam in Verhältnissen von 1:10 bis 10:1, bevorzugt 3:1 bis 1:3 und ganz besonders bevorzugt in stöchiometrischen Mengen, eingesetzt.

Auch protische Lösemittel, wie beispielsweise Wasser, und Alkohole können in den erfindungsgemäßen Zusammensetzungen enthalten sein. Als Alkohole finden alle physiologisch bedenkenlos verwendbaren Alkohole Verwendung, beispielsweise Methanol, Ethanol, Isopropanol, Propanol, Butanol, Isobutanol, Glykol, Glycerin und deren Mischungen untereinander. Der Anteil an protischen Lösemitteln ergänzt in jedem Fall die erfindungsgemäße Zusammensetzung auf 100 Gewichtsteile. Bevorzugt sind in den kosmetischen Zusammensetzungen mindestens 30 Gew.% protische Lösemittel, besonders bevorzugt mindestens 50 Gew.% und ganz besonders bevorzugt mindestens 75 Gew.% sowie höchst bevorzugt mindestens 85 Gew.% protische Lösemittel enthalten.

Weiterhin können in einer ganz besonders bevorzugten Ausführungsform der Erfindung die UV-Filter (I) verwendet werden. Die erfindungsgemäß zu verwendenden UV-Filter unterliegen hinsichtlich ihrer Struktur und ihrer physikalischen Eigenschaften keinen generellen Einschränkungen. Vielmehr eignen sich alle im Kosmetikbereich einsetzbaren UV-Filter, deren Absorptionsmaximum im UVA(315-400 nm)-, im UVB(280-315nm)- oder im UVC(<280 nm)-Bereich liegt. UV-Filter mit einem Absorptionsmaximum im UVB-Bereich, insbesondere im Bereich von etwa 280 bis etwa 300 nm, sind besonders bevorzugt.

Die UV-Filter (I) sind in den erfindungsgemäß verwendeten Mitteln üblicherweise in Mengen 0,1-5 Gew.-%, bezogen auf das gesamte Mittel, enthalten. Mengen von 0,4-2,5 Gew.-% sind bevorzugt. Weitere Wirk-, Hilfs- und Zusatzstoffe sind beispielsweise
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- haarkonditionierende Verbindungen wie Phospholipide, beispielsweise Sojalecithin, Ei-Lecitin und Kephaline,
- Dimethylisosorbid und Cyclodextrine,
- symmetrische und unsymmetrische, lineare und verzweigte Dialkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether und Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether,
- faserstrukturverbessernde Wirkstoffe, insbesondere Mono-, Di- und Oligosaccharide, wie beispielsweise Glucose, Galactose, Fructose, Fruchtzucker und Lactose,
- Phospholipide, beispielsweise Sojalecithin, Ei-Lecithin und Kephaline,
- quaternierte Amine wie Methyl-1-alkylamidoethyl-2-alkylimidazolinium-methosulfat,
- Antischuppenwirkstoffe wie Piroctone Olamine, Zink Omadine und Climbazol,
- Wirkstoffe wie Allantoin und Bisabolol,
- Cholesterin,
- Komplexbildner wie EDTA, NTA, β-Alanindiessigsäure, Iminodibersnsteinsäure und derne Salze, Etidronic acid und deren Salze und Phosphonsäuren,
- Quell- und Penetrationsstoffe wie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Reduktionsmittel wie z. B. Thioglykolsäure und deren Derivate, Thiomilchsäure, Cysteamin, Thioäpfelsäure und a-Mercaptoethansulfonsäure,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. die Monographie von K. H. Schrader verwiesen.

Ein weiterer Gegenstand der vorliegenden Erfindung ist ein Verfahren zur Haarbehandlung, bei dem die erfindungsgemäße Zusammensetzung in das feuchte Haar eingearbeitet wird, und die Zusammensetzung nach einer Einwirkzeit von wenigen Sekunden bis zu 45 Minuten mit kaltem bis zu etwa 40 °C warmem Wasser wieder ausgespült wird, gegebenenfalls das Haar mit einem Handtuch getrocknet wird, dann eine weitere pflegende und konditionierende Zusammensetzung B in das Handtuch trockene bis nasse Haar eingearbeitet wird, wobei diese Zusammensetzung wenige Sekunden bis hin zu 30 Minuten auf dem Haar verbleibt, und anschließend ausgespült wird, das Haar wiederum mit einem Handtuch getrocknet wird, gegebenenfalls gefolgt von einem Föhnschritt, um das Haar vollständig zu trocknen, und letztlich eine frisierende und stylende Zusammensetzung C in das Haar gegeben wird und vorzugsweise nicht wieder ausgespült wird. Sowohl die Zusammensetzung B als pflegende und konditionierende Zusammensetzung als auch die Zusammensetzung C können selbstverständlich wiederum die erfindungsgemäße Zusammensetzung enthalten. Bevorzugt ist es, wenn mindestens die Zusammensetzung B ebenfalls die erfindungsgemäße Zusammensetzung enthält.

Das erfindungsgemäße Verfahren umfasst auch, dasa der erste Schritt der Haarwäsche gegebenenfalls wiederholt werden kann.

### Beispiele und Wirkungsnachweise

Alle Mengenangaben sind, soweit nicht anders vermerkt, Gewichtsteile.

### 1. Wirkungsnachweis

### Vorbehandlung

Strähnen der Fa. Alkinco (1,0 g, Code 6634) wurden mit 2,0 g einer Lösung aus 10,0 Gew. % Natriumlaurylethersulfat (2EO) für 2 Minuten shampooniert. Anschließend wurde 1 Minute unter fließendem Wasser von 23 °C ausgespült und die Haarsträhne mit einem Haarföhn getrocknet. Es folgte eine herkömmliche Dauerwellbehandlungen mit 2,0 g des Handelsproduktes Poly Lock-Normale Dauerwelle. Im Rahmen dieser Dauerwellbehandlung wurden die Fasern in einem ersten Schritt für 40 Minuten bei Raumtemperatur der Reduktionslösung (enthaltend 7,9 Gew.-% Thioglykolsäure) ausgesetzt, mit reinem Wasser mit einer Temperatur von 23 °C für 1 Minute unter fließendem Wasser gespült, mit einem Handtuch getrocknet und anschließend bei Raumtemperatur für 10 Minuten mit 2,0 g einer Oxidationslösung fixiert. Die Oxidationslösung enthielt 2,6 Gew.-% Wasserstoffperoxid. Nach der oxidativen Behandlung wurden die Fasern wiederum für 1 Minute unter fließendem Wasser bei 23 °C gespült und anschließend mit einem Haarföhn getrocknet. Die Dauerwellbehandlung wurde insgesamt unter identischen Bedingungen zweimal durchgeführt.

### a) Nachbehandlung

Die Strähnen wurden jeweils bei einer Temperatur von 32°C 30 Minuten in eine 1 % ige wäßrige Lösung der jeweiligen Wirkstoffe mit 0,2 Gew.% Ceteareth-25 als Emulgator bei einem pH - Wert von 7, welcher mit Natronlauge oder Salzsäure eingestellt wurde, getaucht. Im Falle des Wirkstoffkomplexes aus Arganöl und Sheabutter wurden die Mengen der einzelnen Wirkstoff in Gew.% vermerkt und in jedem Falle mit 0,2 Gew.% Ceteareth-25 als Emulgator versetzt. Nach der Behandlung mit der jeweiligen Wirkstoffzusammensetzung wurde jede Haarsträhne 1 Minute mit klarem Wasser gespült, an der Luft getrocknet und 16 h ruhen gelassen.

### b) Nachweis der haarstrukturierenden Wirkung mittels HP-DSC

Mittels einer DSC-Analyse (Perkin Elmer DSC-7) wurden die folgenden in Tabelle 1 dargestellten Schmelzpunkte ermittelt. Eine genaue Beschreibung der Methode findet sich beispielsweise in der DE 196 173 95 A1

| Wirkstoff bzw. Wirkstoffkomplex | Schmelzpunkt in °C |
|---|---|
| 1. Ohne Wirkstoff | 149,0 |
| 2. Arganöl (1,0 Gew.%) | 152,3 |
| 3. Sheabutter (1,0Gew.%) | 152,1 |
| 4. Polyquaternium-10 (0,1 Gew.%) | 149,7 |
| 5. Arganöl (0,5 Gew.%) und Sheabutter (0,5 Gew.%) | 155,2 |
| 6. Arganöl (0,8 Gew.%) und Sheabutter (0,1 Gew.%) und Polyquaternium-10 (0,1 Gew. %) | 158,0 |
| 7. Arganöl (0,6 Gew.%) und Sheabutter (0,3 Gew.%) und Polyquaternium-10 (0,1 Gew. %) | 159,1 |

Das Signifikanzniveau der Wirkstoffkomplexe 5 bis 7 gegenüber dem Wert ohne Wirkstoff ist >99%.

Die Wirkstoffkomplexe aus der obigen Tabelle (die laufenden Nummern 5 bis 7 gegen die Lösungen der laufenden Nummern 1 bis 4) wurden weiterhin im Halbseitentest untersucht. Dazu wurden 40 Testpersonen mit unterschiedlichen Haargrundlagen (gefärbt, normal, dauergewellt, blondiert, dauergewellt und blondiert etc.) mit jeweils 10,0 g einer Lösung aus 10,0 Gew. % Natriumlaurylethersulfat (2EO) für 2 Minuten die Haare gewaschen und mit dem Handtuch getrocknet. Das so vorbereitete Haar wurde in der Mitte gescheitelt. Auf jede Hälfte wurden 2,5 g der jeweiligen Testlösungen als Non-Aerosol aufgesprüht. Nach 3 Minuten wurden die Haare erneut für 2 Minuten gewaschen. Anschließend wurde das nasse und nach dem Trocknen mit einem Haarföhn das trockene Haar von jeweils 4 Friseurfachkräften beurteilt. Die Zusammensetzungen der Wirkstoffkomplexe mit den laufenden Nummern 5 bis 7 wurden in den Nass- und Trockenkämmbarkeiten, sowie dem Griff im nassen und trockenen Haar, dem Volumen und dem Glanz deutlich besser beurteilt. Weiterhin zeigte sich bei diesen Wirkstoffkomplexen eine deutlich lang anhaltende Wirkung in bezug auf das Volumen, den Glanz und die Gestaltbarkeit der Frisur und den Halt der Frisur.

### 2. Weitere Formulierungsbeispiele:

**Shampoo:**

| | |
|---|---|
| Citronensäure | 0,5 |
| Sodium Laureth Sulfate | 13,0 |
| Disodium Cocoamphodiacetate | 6,0 |
| Salicylsäure | 0,2 |
| D-Panthenol (75%) | 0,2 |
| Sodium Benzoate | 0,5 |
| Euperlan PK 3000 AM | 2,6 |
| Cetiol HE | 1,5 |
| Hydrogenated Castor Oil | 0,5 |
| Arganöl | 0,4 |
| Sheabutter | 0,1 |
| Ceteareth-25 | 0,5 |
| Sodium Chloride | 0,5 |
| Wasser | Ad 100 |

**Haarkur:**

| | |
|---|---|
| Paraffinum Liquidum | 1,5 |
| Dehyquart F 75 | 1,5 |
| Isopropylmyristat | 1,0 |
| Varisoft W 75 PG | 1,5 |
| Cetearyl Alcohol | 4,0 |
| Propylparaben | 0,2 |
| Arganöl | 1,0 |
| Ceteareth-9 | 0,8 |
| Stearamidopropyldimethylamine | 0,8 |
| Cetyltrimethylammoniumchlorid | 0,75 |
| Citronensäure, wasserfrei | 0,4 |
| Methylparaben | 0,2 |
| Phenoxyethanol | 0,2 |
| Sheabutter | 0,2 |
| Salcare SC 96 | 0,5 |
| Wasser | Ad 100 |

**Haarspülung:**

| | |
|---|---|
| Stenol 1618 | 7,0 |
| Sheabutter | 0,15 |
| Genamin KDM-P | 1,2 |
| Dehyquart F 75 | 1,2 |
| Arganöl | 0,5 |
| Ceteareth-25 | 0,8 |
| Cetylester | 0,5 |
| Methylparaben | 0,2 |
| Parfüm | 0,3 |
| Phenoxyethanol | 0,4 |
| Wasser | Ad 100 |

Diese Haarspülung ist auch hervorragend geeignet, um als Nachbehandlungsmittel im Anschluss an ein oxidatives Färbeverfahren verwendet zu werden. Das hiermit nachbehandelte gefärbte Haar zeigt einen deutlichen und intensiven gleichmäßigen Glanz bei einer sehr guten Kämmbarkeit und angenehmen Griffeigenschaften im nassen und trockenen Haar. Weiterhin lässt es sich sehr gut mit Stylingmitteln frisieren und zu einer Frisur formen.

**Haartonic**

| | |
|---|---|
| Sheabutter | 0,01 |
| Allantoin | 0,1 |
| Benzophenone-4 | 0,03 |
| Synthalen K | 0,24 |
| Neutrol TE | 0,25 |
| Ethanol 96% | 40,0 |
| Arganöl | 0,08 |
| Ceteareth-25 | 0,1 |
| Menthol | 0,03 |
| Wasser | Ad 100 |

### Oxidative Haarfärbecremes:

Die im Folgenden beschriebenen Färbecremes I bis XVI (Tabellen 1-4) wurden hergestellt und jeweils unmittelbar vor der Anwendung im Verhältnis 1:1 mit jeweils einer der folgenden beiden Oxidationsmittelzubereitungen vermischt:

| Rohstoff | Ox 1 | Ox 2 |
|---|---|---|
| Dipicolinsäure | 0,1 | 0,1 |
| 50%ige KOH | 0,3 | 0,3 |
| Natriumbenzoat | 0,04 | 0,04 |
| Natriumpyrophosphat | 0,1 | 0,1 |
| Turpinal^{®} SL | 0,4 | 0,4 |
| 1,2-Propylenglykol | 0,4 | 0,4 |
| Cetyl/Stearylalkohol 50 : 50 | 4,0 | 4,0 |
| Dehyquart^{®} B | 0,75 | 0,75 |
| Emulgin^{®} B2 | 1,2 | 1,2 |
| Paraffinöl | 0,3 | -------- |
| Sheabutter | -------- | 0,03 |
| Arganöl | -------- | 0,1 |
| 50%ige H₂O₂ | 12,2 | 12,2 |
| Wasser | ad 100,0 | ad 100,0 |

Die resultierende Anwendungsmischung wurde auf Strähnen aus Büffelbauchhaar aufgetragen und dort für 30 Minuten bei Raumtemperatur belassen. Anschließend wurden die Fasern gründlich mit Wasser gespült und die Färbungen ermittelt. Weiterhin wurden die Kämmbarkeiten der nassen und trockenen Haarsträhnen sowie deren Griff und Glanz beurteilt.

### Färbecremes:

**Tabelle 1:**

| Rohstoff | Färbecreme I | Färbecreme II | Färbecreme III | Färbecreme IV |
|---|---|---|---|---|
| Ammoniumcarbopollösung (1% in Wasser) | 15,0 | 15,0 | 15,0 | 15,0 |
| Lanette^{®} E | 0,7 | 0,7 | 0,7 | 0,7 |
| Laurylethersulfat (27% Aktivsubstanz) | 4,4 | 4,4 | 4,4 | 4,4 |
| PEG 400 | 0,6 | 0,6 | 0,6 | 0,6 |
| Potassium Oleate (12.5% Aktivsubstanz) | 3,0 | 3,0 | 3,0 | 3,0 |
| Titanium Dioxide | 0,5 | 0,5 | 0,5 | 0,5 |
| Cetylstearylalcohol 50/50 | 12,0 | 12,0 | 12,0 | 12,0 |
| Eumulgin^{®} B2 | 3,0 | 3,0 | 3,0 | 3,0 |
| Eutanol^{®} G | 2,0 | 2,0 | 2,0 | 2,0 |
| Cutina^{®} AGS | 2,0 | 2,0 | 2,0 | 2,0 |
| Cutina^{®} GMS-SE2,002,02,01,0 | | | | |
| Kalilauge 50% | 0,9 | 0,9 | 0,9 | 0,9 |
| Tetrasodium EDTA | 0,2 | 0,2 | 0,2 | 0,2 |
| Sodium Sulfite | 0,2 | 0,2 | 0,2 | 0,2 |
| Ascorbic Acid | 0,05 | 0,05 | 0,05 | 0,05 |
| Merquat Plus^{®} 3330 | 1,5 | 1,5 | 1,5 | 1,5 |
| Parfum | 0,3 | 0,3 | 0,3 | 0,3 |
| Phospholipid^{®} EFA | 0,1 | 0,1 | 0,1 | 0,1 |
| Puricare^{®} LS 9658 | 1,0 | 1,0 | 1,0 | 1,0 |
| Sheabutter | 0,05 | 0,05 | 0,05 | 0,05 |
| Arganöl | 0,1 | 0,1 | 0,1 | 0,1 |
| Kieselsäure | 0,25 | 0,25 | 0,25 | 0,25 |
| p-Aminophenol | 0,02 | 0,2 | -- | -- |
| p-Phenylendiamine | 1,15 | -- | -- | -- |
| 1-(2-Hydroxyethyl)-4,5-diaminopyrazol | -- | -- | 1,5 | 0,9 |
| p-Toluylene diamine | -- | 0,97 | 0,18 | -- |
| HC Blue No. 7 | -- | -- | -- | 0,40 |
| Resorcinol | 0,60 | 0,45 | -- | -- |
| o-Am inophenol | 0,06 | -- | -- | -- |
| m-Aminophenol | 0,15 | 0,09 | 0,04 | -- |
| 2,6 Diaminopyridin | 0,01 | -- | -- | -- |
| 5-Amino-2-methylphenol | -- | 0,06 | 0,80 | -- |
| 1-Naphthol | -- | -- | -- | 0,40 |
| 2-Amino-4-Hydroxyethylaminoanisole Sulfat | -- | -- | -- | 0,13 |
| 2-Nitro-p-Phenylenediamine | -- | 0,20 | -- | -- |
| Ammonia Solution (25%) | ad pH 10,2 | ad pH 10,4 | ad pH 10,2 | ad pH 10,5 |
| Wasser | ad 100,0 | ad 100,0 | ad 100,0 | ad 100,0 |
| Nuance | Hellbraun | Kupfer | Rot | Violett |

**Tabelle 2:**

| Rohstoff | Färbecreme V | Färbecreme VI | Färbecreme VII | Färbecreme VIII |
|---|---|---|---|---|
| Ammoniumcarbopollösung (1% in Wasser) | 18,0 | 18,0 | 18,0 | 18,0 |
| Ammonium Rohagit^{®} (6% in Wasser) | 15,0 | 15,0 | 15,0 | 15,0 |
| Eumulgin^{®} KE 2602 | 0,7 | 0,7 | 0,7 | 0,7 |
| Potasium Oleate | 4,4 | 4,4 | 4,4 | 4,4 |
| Potassium Castorate | 0,6 | 0,6 | 0,6 | 0,6 |
| Plantacare^{®} 2000 UP | 3,0 | 3,0 | 3,0 | 3,0 |
| Titaniumdioxide | 0,5 | 0,5 | 0,5 | 0,5 |
| Lanette^{®} O | 12,0 | 12,0 | 12,0 | 12,0 |
| Cetiol^{®} V | 3,0 | 3,0 | 3,0 | 3,0 |
| Cutina^{®} GMS V | 2,0 | 2,0 | 2,0 | 2,0 |
| Kalilauge 50% | 0,9 | 0,9 | 0,9 | 0,9 |
| Tetrasodium EDTA | 0,2 | 0,2 | 0,2 | 0,2 |
| Sodium Sulfite | 0,2 | 0,2 | 0,2 | 0,2 |
| Ascorbic Acid | 0,05 | 0,05 | 0,05 | 0,05 |
| Mirapol^{®} A 15 | 1,5 | 1,5 | 1,5 | 1,5 |
| Parfum | 0,3 | 0,3 | 0,3 | 0,3 |
| Phospholipid^{®} EFA | 0,1 | 0,1 | 0,1 | 0,1 |
| Puricare^{®} LS 9658 | 1,0 | 1,0 | 1,0 | 1,0 |
| Sheabutter | 0,05 | 0,05 | 0,05 | 0,05 |
| Arganöl | 0,1 | 0,1 | 0,1 | 0,1 |
| Kieselsäure | 0,25 | 0,25 | 0,25 | 0,25 |
| p-Aminophenol | 0,02 | 0,2 | -- | -- |
| p-Phenylendiamine | 1,15 | -- | -- | -- |
| 1-(2-Hydroxyethyl)-4,5-diaminopyrazol | -- | -- | 1,5 | 0,93 |
| p-Toluylenediamine | -- | 0,97 | 0,18 | -- |
| HC Blue No. 7 | -- | -- | -- | 0,40 |
| Resorcinol | 0,60 | 0,45 | -- | -- |
| o-Aminophenol | 0,06 | -- | -- | -- |
| m-Aminophenol | 0,15 | 0,09 | 0,04 | -- |
| 2,6 Diaminopyridin | 0,011 | -- | -- | -- |
| 5-Amino-2-methylphenol | -- | 0,06 | 0,80 | -- |
| 1-Naphthol | -- | -- | -- | 0,40 |
| 2-Amino-4-Hydroxyethylaminoanisole sulfat | -- | -- | -- | 0,13 |
| 2-Nitro-p-Phenylenediamine | -- | 0,20 | -- | -- |
| Ammonia Solution (25%) | ad 10,2 | ad 10,4 | ad 10,3 | ad 10,5 |
| Wasser | ad 100,0 | ad 100,0 | ad 100,0 | ad 100,0 |
| Nuance | Hellbraun | Kupfer | Rot | Violett |

**Tabelle 3:**

| Rohstoff | Färbecreme IX | Färbecreme IX | Färbecreme XI | Färbecreme XII |
|---|---|---|---|---|
| Ammoniumcarbopollösung (1 %in Wasser) | 15,0 | 15,0 | 15,0 | 15,0 |
| La nette^{®} E | 0,70 | 0,7 | 0,7 | 0,7 |
| Laurylethersulfat (27% in Wasser) | 4,4 | 4,4 | 4,4 | 4,4 |
| PEG 600 | 0,6 | 0,6 | 0,6 | 0,6 |
| Potassium Oleate (12.5% in Wasser) | 3,0 | 3,0 | 3,0 | 3,0 |
| Titanium Dioxide | 0,15 | 0,15 | 0,15 | 0,150 |
| Cetylstearylalcohol 50/50 | 12,0 | 12,0 | 12,0 | 12,0 |
| Eumulgin^{®} B2 | 3,0 | 3,0 | 3,0 | 3,0 |
| Eutanol^{®} G | 2,0 | 2,0 | 2,0 | 2,0 |
| Cutina^{®} AGS | 2,0 | 2,0 | 2,0 | 2,0 |
| Cutina^{®} GMS-SE | 2,0 | 2,0 | 2,0 | 2,0 |
| XF42-B1989^{®} | 1,5 | 1,5 | 1,5 | 1,5 |
| Glycyrrhiza Extracted Powder | 0,2 | 0,2 | 0,2 | 0,2 |
| Kalilauge 50% | 0,9 | 0,9 | 0,9 | 0,9 |
| Tetrasodium EDTA | 0,4 | 0,4 | 0,4 | 0,4 |
| Sodium Sulfite | 0,1 | 0,1 | 0,1 | 0,1 |
| Ascorbic Acid | 0,1 | 0,1 | 0,1 | 0,1 |
| Merquat Plus^{®} 3330 | 1,5 | 1,5 | 1,5 | 1,5 |
| Parfum | 0,5 | 0,5 | 0,5 | 0,5 |
| Puricare^{®} LS 9658 | 1,0 | 1,0 | 1,0 | 1,0 |
| Sheabutter | 0,05 | 0,05 | 0,05 | 0,05 |
| Arganöl | 0,1 | 0,1 | 0,1 | 0,1 |
| Kieselsäure | 0,25 | 0,25 | 0,25 | 0,25 |
| p-Aminophenol | 0,02 | 0,20 | -- | -- |
| p-Phenylendiamine | 1,15 | -- | -- | -- |
| 1-(2-Hydroxyethyl)-4,5-diaminopyrazol | -- | -- | 1,50 | 0,93 |
| p-Toluylenediamine | -- | 0,97 | 0,18 | -- |
| HC Blue No. 7 | -- | -- | -- | 0,40 |
| Resorcinol | 0,60 | 0,45 | -- | -- |
| o-Am inophenol | 0,06 | -- | -- | -- |
| m-Aminophenol | 0,15 | 0,09 | 0,04 | -- |
| 2,6 Diaminopyridin | 0,01 | -- | -- | -- |
| 5-Amino-2-methylphenol | -- | 0,06 | 0,80 | -- |
| 1-Naphthol | -- | -- | -- | 0,40 |
| 2-Amino-4-Hydroxyethylaminoanisole Sulf. | -- | -- | -- | 0,13 |
| 2-Nitro-p-Phenylenediamine | -- | 0,20 | -- | -- |
| Ammonia Solution (25%) | ad 10,2 | ad 10,4 | ad 10,3 | ad 10,5 |
| Wasser | ad 100,0 | ad 100,0 | ad 100,0 | ad 100,0 |
| Nuance | Hellbraun | Kupfer | Rot | Violett |

**Tabelle 4:**

| Rohstoff | Färbecreme XIII | Färbecreme XIV | Färbecreme XV | Färbecreme XVI |
|---|---|---|---|---|
| Ammoniumcarbopollösung (1% in Wasser) | 15,0 | 15,0 | 15,0 | 15,0 |
| Lanette^{®} E | 0,7 | 0,7 | 0,7 | 0,7 |
| Laurylethersulfat (27% in Wasser) | 4,4 | 4,4 | 4,4 | 4,4 |
| PEG 600 | 0,6 | 0,6 | 0,6 | 0,6 |
| Potassium Oleate (12.5% in Wasser) | 3,0 | 3,0 | 3,0 | 3,0 |
| Titanium Dioxide | 0,15 | 0,15 | 0,15 | 0,15 |
| Cetylstearylalcohol 50/50 | 12,0 | 12,0 | 12,0 | 12,0 |
| Eumulgin^{®} B2 | 3,0 | 3,0 | 3,0 | 3,0 |
| Eutanol^{®} G | 2,0 | 2,0 | 2,0 | 2,0 |
| Cutina^{®} AGS | 2,0 | 2,0 | 2,0 | 2,0 |
| Cutina^{®} GMS-SE | 2,0 | 2,0 | 2,0 | 2,0 |
| XF42-B1989^{®} | 1,5 | 1,5 | 1,5 | 1,5 |
| Glycyrrhiza Extracted Powder | 0,2 | 0,2 | 0,2 | 0,2 |
| Potassium Carbonate, Anhydrous | 1,0 | 1,0 | 1,0 | 1,0 |
| Diammoniumhydrogenphosphate | 0,6 | 0,6 | 0,6 | 0,6 |
| Tetrasodium EDTA | 0,4 | 0,4 | 0,4 | 0,4 |
| Sodium Sulfite | 0,1 | 0,1 | 0,1 | 0,1 |
| Ascorbic Acid | 0,1 | 0,1 | 0,1 | 0,1 |
| Merquat Plus^{®} 3330 | 1,5 | 1,5 | 1,5 | 1,5 |
| Parfum | 0,5 | 0,5 | 0,5 | 0,5 |
| Puricare^{®} LS 9658 | 1,0 | 1,0 | 1,0 | 1,0 |
| Sheabutter | 0,05 | 0,05 | 0,05 | 0,05 |
| Arganöl | 0,1 | 0,1 | 0,1 | 0,1 |
| Kieselsäure | 0,25 | 0,25 | 0,25 | 0,25 |
| p-Aminophenol | 0,02 | 0,20 | -- | -- |
| p-Phenylendiamine | 1,15 | -- | -- | -- |
| 1-(2-Hydroxyethyl)-4,5-diaminopyrazol | -- | -- | 1,50 | 0,93 |
| p-Toluylene diamine | -- | 0,97 | 0,18 | -- |
| HC Blue No. 7 | -- | -- | -- | 0,40 |
| Resorcinol | 0,60 | 0,45 | -- | -- |
| o-Am inophenol | 0,06 | -- | -- | -- |
| m-Aminophenol | 0,15 | 0,09 | 0,04 | -- |
| 2,6 Diaminopyridin | 0,01 | -- | -- | -- |
| 5-Amino-2-methylphenol | -- | 0,06 | 0,80 | -- |
| 1-Naphthol | -- | -- | -- | 0,40 |
| 2-Amino-4-Hydroxyethylaminoanisole Sulfat | -- | -- | -- | 0,13 |
| 2-Nitro-p-Phenylenediamine | -- | 0,20 | -- | -- |
| Kalilauge (50% in Wasser) | ad pH 9,1 | ad pH 9,1 | ad pH 9,1 | ad pH 9,1 |
| Wasser | ad 100,0 | ad 100,0 | ad 100,0 | ad 100,0 |
| Nuance | Hellbraun | Kupfer | Rot | Violett |

In allen Fällen waren die gefärbten Strähnen im nassen und trockenen Haar sehr leicht bis leicht kämmbar, überzeugten durch einen angenehmen, weichen Griff sowie einer intensiven, glänzenden und gleichmäßigen Färbung.

### Verzeichnis der eingesetzten Handelsprodukte

Die im Rahmen der Beispiele eingesetzten Handelsprodukte sind wie folgt definiert:

| | |
|---|---|
| Ammoniumcarbopollsg. | Lösung eines Ammoniumsalzes eines Methacrylsäure-Methylacrylat-Copolymeren (INCI-Bezeichnung: Ammonium Polyacrylate) (Röhm GmbH) |
| Ammoniumrohagitlösung | Lösung eines Ammoniumsalzes eines Acrylsäure-Polymeren (INCI-Bezeichnung: Ammonium Acrylates Copolymer) (Goodrich) |
| Cetiol^{®} V | Ölsäuredecylester (INCI-Bezeichnung: Decyl Oleate) (Cognis) |
| Cutina^{®} AGS | Ethylenglykoldistearat (INCI-Bezeichnung: Glycol Distearate) (Cognis) |
| Cutina^{®} GMS-SE | INCI-Bezeichnung: Glyceryl Stearate SE (Cognis) |
| Cutina^{®} GMS-V | Glycerinmono/dipalmitat/stearat (INCI-Bezeichnung: Glyceryl Stearate) (Cognis) |
| Dehyquart^{®} B | Stearyltrimethylammoniumchlorid (ca. 60-66% Aktivsubstanzgehalt; INCI-Bezeichnung: Steartrimonium Chloride) (Cognis) |
| Eumulgin^{®} B2 | Cetylstearylalkohol mit ca. 20 EO-Einheiten (INCI-Bezeichnung: Ceteareth-20) (Cognis) |
| Eumulgin^{®} KE 2602 | ethoxylierter Oleylalkohol (INCI-Bezeichnung: Oleth-7) (Cognis) |
| Eutanol^{®} G | 2-Octyldodecylalkohol (INCI-Bezeichnung: Octyldodecanol) (Cognis) |
| Glycyrrhiza Extr. Powder | Licorice-Extrakt (INCI-Bezeichnung: Glycyrrhiza Glabra (Licorice) Root Extract) (Maruzen) |
| HC Blue No.7 | INCI-Bezeichnung: 6-Methoxy-2-methylamino-3-aminopyridine HCl |
| Lanette^{®} E | Fettalkoholsulfat-Natrium-Salz (ca. 90-96% Aktivsubstanz-gehalt; INCI-Bezeichnung: Sodium Cetearyl Sulfate) (Cognis) |
| Lanette^{®} O | C₁₆₋₁₈-Fettalkohol (INCI-Bezeichnung: Cetearyl Alcohol) (Cognis) |
| Merquat^{®} Plus 3330 | Dimethyldiallylammoniumchlorid Acrylsäure Acrylamid Terpolymer (ca. 9,5% Festkörper in Wasser; INCI-Bezeichnung: Polyquaternium-39) (Ondeo-Nalco) |
| Mirapol^{®} A 15 | Poly[N-(3-(dimethylammonium)propyl]-N'-[3-ethylenoxyethylendimethyl-ammonium)-propyl]-harnstoff-di-chlorid (ca. 64% Festkörper in Wasser; INCI-Bezeichnung: Polyquaternium-2) (Rhodia) |
| Phopholipid^{®} EFA | (ca. 30% Festkörper in Wasser/Propylenglykol; INCI-Bezeichnung:Linoleamidopropyl PG-Dimonium Chloride Phosphate) (Uniqema) |
| Plantacare^{®} 2000 UP | C₈₋₁₆ Alkylglucosid (ca. 51-55% Aktivsubstanzgehalt in Wasser; INCI-Bezeichnung: Decyl Glucoside, Aqua (Water)) (Cognis) |
| Puricare^{®} LS 9658 | Moringa Pterygosperma Extrakt, in Wasser/Glycerin (ca. 1%ige Lösung; INCI-Bezeichnung: Water, Glycerine, Moringa Pterygosperma Seed Extract) (Cognis) |
| Turpinal^{®} SL | 1-Hydroxyethan-1,1-diphosphonsäure (ca. 58 - 61% Aktivsubstanzgehalt; INCI-Bezeichnung: Etidronic Acid, Aqua (Water)) (Solutia) |
| XF42-B1989^{®} | aminofunktionelles Silikon (INCI-Bezeichnung: Amodimethicone) (GE Bayer Silicones) |

## Patentansprüche

1. Verfahren zur Behandlung keratinischer Fasern, **dadurch gekennzeichnet, dass** eine kosmetische Zusammensetzung enthaltend einen Wirkstoffkomplex enthaltend Arganöl und Sheabutter auf die keratinischen Fasern aufgetragen wird und nach einer Einwirkzeit von wenigen Sekunden bis hin zu 45 Minuten wieder ausgespült wird.

2. Verfahren zur Behandlung keratinischer Fasern nach Anspruch 1, **dadurch gekennzeichnet, dass** die kosmetische Zusammensetzung enthaltend einen Wirkstoffkomplex enthaltend Arganöl und Sheabutter weiterhin mindestens einen weiteren Wirkstoff ausgewählt aus den kationischen und/oder amphoteren und/oder zwitterionischen Polymeren enthält.

3. Verfahren zur Behandlung keratinischer Fasern nach einem der Ansprüche 1 oder 2, **dadurch gekennzeichnet, dass** die kosmetische Zusammensetzung enthaltend einen Wirkstoffkomplex enthaltend Arganöl und Sheabutter weiterhin einen Fettstoff (D) enthält.

4. Verfahren zur Behandlung keratinischer Fasern nach einem der Ansprüche 1 bis 3, **dadurch gekennzeichnet, dass** der Fettstoff (D) ausgewählt ist aus den Silikonen (S).

## Claims

1. A method for treating keratin fibers, **characterized in that** a cosmetic composition containing an active ingredient complex containing argan oil and shea butter is applied to the keratin fibers and rinsed out again after an application time of from a few seconds up to 45 minutes.

2. The method for treating keratin fibers according to claim 1, **characterized in that** the cosmetic composition containing an active ingredient complex containing argan oil and shea butter further contains at least one additional active ingredient selected from the cationic and/or amphoteric and/or zwitterionic polymers.

3. The method for treating keratin fibers according to one of claims 1 or 2, **characterized in that** the cosmetic composition containing an active ingredient complex containing argan oil and shea butter further contains a fatty substance (D).

4. The method for treating keratin fibers according to one of claims 1 to 3, **characterized in that** the fatty substance (D) is selected from the silicones (S).

## Revendications

1. Procédé de traitement des fibres de kératine, **caractérisé en ce qu'**une composition cosmétique contenant un complexe d'agents actifs contenant de l'huile d'argan et du beurre de karité est appliquée sur les fibres de kératine et est éliminée par rinçage après un temps d'action de quelques secondes à 45 minutes.

2. Procédé de traitement des fibres de kératine selon la revendication 1, **caractérisé en ce que** la composition cosmétique contenant un complexe d'agents actif contenant de l'huile d'argan et du beurre de karité contient en outre au moins un autre agent actif choisi parmi les polymères cationiques et/ou amphotères et/ou zwittérioniques.

3. Procédé de traitement des fibres de kératine selon l'une des revendications 1 ou 2, **caractérisé en ce que** la composition cosmétique contenant un complexe d'agents actif contenant de l'huile d'argan et du beurre de karité contient en outre un matière grasse (D).

4. Procédé de traitement des fibres de kératine selon l'une des revendications 1 à 3, **caractérisé en ce que** la matière grasse (D) est choisie parmi les silicones (S).
